# EUROPEAN PATENT APPLICATION

(11) **EP 1 734 125 A1**
(43) Date of publication of application: **20.12.2006**
(21) Application number: 05291294.6
(22) Date of filing: 16.06.2005
(51) Int. Cl.: C12N 15/82, C12Q 1/68

(54) **Homeologous recombination in MSH2 inactivated plants or cells thereof**

(71) Applicant: INSTITUT NATIONAL DE LA RECHERCHE AGRONOMIQUE, 75007 Paris (FR); CELLECTIS, 93235 Romainville Cédex (FR)
(72) Inventor: Nogue, Fabien, 78910 Orgerus (FR); Trouiller, Bénédicte, 92120 Montrouge (FR); Charlot, Florence, 75002 Paris (FR)
(74) Representative: Vialle-Presles, Marie José

(57) **Abstract**

A method for producing a hybrid or transgenic plant, or cell thereof, comprising at least the steps of: a) inactivating transitorily MSH2 in a plant or cell thereof, b) introducing in said plant or cell thereof, a targeting sequence having from 0.1 % to 10 % differences with a target sequence in the genome of said plant or cell thereof, under conditions allowing homeologous recombination to occur between said target and targeting sequences, and c) identifying the hybrid or transgenic plant or cell thereof, in which genomic integration of said targeting sequence as occurred through homeologous recombination with said target sequence.

SEQUENCE LISTING

<110>  INSTITUT NATIONAL DE LA RECHERCHE AGRONOMIQUE
       CELLECTIS

<120>  Homeologous recombination in MSH2 inactivated plants or cells
       thereof

<130>  539EP129

<160>  9

<170>  PatentIn version 3.3

<210>  1
<211>  3308
<212>  DNA
<213>  Physcomitrella patens

<220>
<221>  CDS
<222>  (246)..(3086)

<400>  1
gttgagtcga atttgcgaag ccgtcgactg gagcgagatt gtgttagcgc cgctctggtc      60

agccttcttc atactacacc tcggtgagtt cgggatttgg gagcgcaatt tcgtaggagc     120

ttgaagtatt ttcgcttttc gagtgctttg agcaccgagg cgttgcgttt ttttgaagat     180

ttgaatacct acagcatagt tagtgttgga tttggcaatc cgattgtggg atggacccag     240

atagt atg gag aag ggg gat gag ccg aag ctg ccc gaa ttg aag cta gat     290
      Met Glu Lys Gly Asp Glu Pro Lys Leu Pro Glu Leu Lys Leu Asp
      1               5                   10                  15

gtt aag cag gca cag ggg ttc att tct ttc ttc cgc agg ctt ccc gag       338
Val Lys Gln Ala Gln Gly Phe Ile Ser Phe Phe Arg Arg Leu Pro Glu
                20                  25                  30

aac gag caa gct gtg cgc ttc ttt gac cgc agg gat tat ttc act gct       386
Asn Glu Gln Ala Val Arg Phe Phe Asp Arg Arg Asp Tyr Phe Thr Ala
            35                  40                  45

cat ggt gat aat gca acc ttc att gcc agt acg tac tat agg acg ctc       434
His Gly Asp Asn Ala Thr Phe Ile Ala Ser Thr Tyr Tyr Arg Thr Leu
        50                  55                  60

aca gtt ctc agg cag ctg ggg agc ggg gct gat tct ctc cct ggt gtg       482
Thr Val Leu Arg Gln Leu Gly Ser Gly Ala Asp Ser Leu Pro Gly Val
    65                  70                  75

acc atc aac aaa agc atg ttt gag act att gtg agg gat ctc ttg cta       530
Thr Ile Asn Lys Ser Met Phe Glu Thr Ile Val Arg Asp Leu Leu Leu
80                  85                  90                  95

gaa aga act gat tgc act gtt gaa ctc tat gag ggc acc gga tct aag       578
Glu Arg Thr Asp Cys Thr Val Glu Leu Tyr Glu Gly Thr Gly Ser Lys
                100                 105                 110

tgg caa ctt gtt agg agc ggg tct cct ggc aag ctt ggc agc ttt gag       626
Trp Gln Leu Val Arg Ser Gly Ser Pro Gly Lys Leu Gly Ser Phe Glu
            115                 120                 125

gag gtc ctg ttc gca agc aat gag atg cag gaa act cct gta gtc atg       674
Glu Val Leu Phe Ala Ser Asn Glu Met Gln Glu Thr Pro Val Val Met

130                 135                 140

gct gta aat att act aca cgt caa agc gag cgg tgt gtt ggg ata gca      722
Ala Val Asn Ile Thr Thr Arg Gln Ser Glu Arg Cys Val Gly Ile Ala
    145                 150                 155

ttc gtg gat atg acg aag agg cag ctt gga atg aca gag ttc tta gac      770
Phe Val Asp Met Thr Lys Arg Gln Leu Gly Met Thr Glu Phe Leu Asp
160                 165                 170                 175

gat gac cta tac acc agt ttg gag tcc gcc atg gta gct tta agc tgt      818
Asp Asp Leu Tyr Thr Ser Leu Glu Ser Ala Met Val Ala Leu Ser Cys
                180                 185                 190

cgt gaa tgt atc ata ccc atg cca acc gct gcc aag tcc cct gat gat      866
Arg Glu Cys Ile Ile Pro Met Pro Thr Ala Ala Lys Ser Pro Asp Asp
            195                 200                 205

cgt aaa ctt cgt gat gta atg gca cgc tgc aat gtt ttg gtt act gag      914
Arg Lys Leu Arg Asp Val Met Ala Arg Cys Asn Val Leu Val Thr Glu
        210                 215                 220

aag aag aaa tct gat ttt agg tcg cga gat gta gaa caa gat tta ggc      962
Lys Lys Lys Ser Asp Phe Arg Ser Arg Asp Val Glu Gln Asp Leu Gly
    225                 230                 235

cga tta gtc aaa ggt cct gct gag caa cat aag gaa ctg gtg gca gca      1010
Arg Leu Val Lys Gly Pro Ala Glu Gln His Lys Glu Leu Val Ala Ala
240                 245                 250                 255

tct gat tta gcg gct gct gct ctt gcc gcg ctt ctg aca tat act gaa      1058
Ser Asp Leu Ala Ala Ala Ala Leu Ala Ala Leu Leu Thr Tyr Thr Glu
                260                 265                 270

ctc ctc tct gat gac aca aat tat gga aaa tac gtc att cag ccc tac      1106
Leu Leu Ser Asp Asp Thr Asn Tyr Gly Lys Tyr Val Ile Gln Pro Tyr
            275                 280                 285

agt ctg gac ctc tac atg cga ttg gat gca gca gct tta cga gct ctc      1154
Ser Leu Asp Leu Tyr Met Arg Leu Asp Ala Ala Ala Leu Arg Ala Leu
        290                 295                 300

aat gtc act gag agc aaa acg gat tct aac aag cat ctt agt ctg ttt      1202
Asn Val Thr Glu Ser Lys Thr Asp Ser Asn Lys His Leu Ser Leu Phe
    305                 310                 315

ggt ttg atg aac cgg act tgc acc caa ggt atg gga aaa aga ttg ctg      1250
Gly Leu Met Asn Arg Thr Cys Thr Gln Gly Met Gly Lys Arg Leu Leu
320                 325                 330                 335

aac cgg tgg ttg aaa caa ccc cta gtg gat gtt gtt gag ata acc aaa      1298
Asn Arg Trp Leu Lys Gln Pro Leu Val Asp Val Val Glu Ile Thr Lys
                340                 345                 350

agg ctg gat aca gtg caa gcg ttt gtg gag gat cta gaa tta agg cag      1346
Arg Leu Asp Thr Val Gln Ala Phe Val Glu Asp Leu Glu Leu Arg Gln
            355                 360                 365

gac ttg cgg tct cac cta cgg cga atg cct gac att gag cgc ttg gtt      1394
Asp Leu Arg Ser His Leu Arg Arg Met Pro Asp Ile Glu Arg Leu Val
        370                 375                 380

agc aag ctt gag aag cgc aaa gcg gga ttg caa gat gtt gtg aga ttg      1442
Ser Lys Leu Glu Lys Arg Lys Ala Gly Leu Gln Asp Val Val Arg Leu
    385                 390                 395

tat cag gcc agt ttg agg ctg cct ttg att cga acc tgc ctg gag aag      1490
Tyr Gln Ala Ser Leu Arg Leu Pro Leu Ile Arg Thr Cys Leu Glu Lys

400                 405                 410                 415

tat gac gga gag ttt tcc aag gat ttg caa gag aaa tac ggc gcc cat      1538
Tyr Asp Gly Glu Phe Ser Lys Asp Leu Gln Glu Lys Tyr Gly Ala His
                420                 425                 430

ctt gat gta tgg aca cag cca aat cat cta gga aaa ttt gat gga tta      1586
Leu Asp Val Trp Thr Gln Pro Asn His Leu Gly Lys Phe Asp Gly Leu
            435                 440                 445

gtc gag gca gct gtc gat ctc gaa cag ctc cag aat gga gag tat ata      1634
Val Glu Ala Ala Val Asp Leu Glu Gln Leu Gln Asn Gly Glu Tyr Ile
        450                 455                 460

tat ttc agc aga tta tgc caa cag tct tca aga gct caa aac tgg gcg      1682
Tyr Phe Ser Arg Leu Cys Gln Gln Ser Ser Arg Ala Gln Asn Trp Ala
    465                 470                 475

cga cga tgt gaa agg cag ata ctt aaa gtt cac cag cag tca gca gat      1730
Arg Arg Cys Glu Arg Gln Ile Leu Lys Val His Gln Gln Ser Ala Asp
480                 485                 490                 495

gac ctg ggg ctt cct gcg gaa aag tca ttg aag ttg gat aag agt aca      1778
Asp Leu Gly Leu Pro Ala Glu Lys Ser Leu Lys Leu Asp Lys Ser Thr
                500                 505                 510

cag tat gga cat gtt ttt agg att act aag aag gaa gag ccg aag gtt      1826
Gln Tyr Gly His Val Phe Arg Ile Thr Lys Lys Glu Glu Pro Lys Val
            515                 520                 525

agg aaa aaa ttg aat agt caa tac ata acc ttg gaa act agg aaa gac      1874
Arg Lys Lys Leu Asn Ser Gln Tyr Ile Thr Leu Glu Thr Arg Lys Asp
        530                 535                 540

ggc ata aag ttc acg aat gcc aag ctg cga agg ttg agt gaa cag tac      1922
Gly Ile Lys Phe Thr Asn Ala Lys Leu Arg Arg Leu Ser Glu Gln Tyr
    545                 550                 555

aca aag ctc aca gag gaa tac tcg agt gca cag cga gag ctt gtt gca      1970
Thr Lys Leu Thr Glu Glu Tyr Ser Ser Ala Gln Arg Glu Leu Val Ala
560                 565                 570                 575

aag gtt gtg gat gtt gca gca aca ttt gtt gag att ttc tca gga gtt      2018
Lys Val Val Asp Val Ala Ala Thr Phe Val Glu Ile Phe Ser Gly Val
                580                 585                 590

gca gtt ctt ctg gct gat atg gat gta ctt ctt agc ttt gct gac tta      2066
Ala Val Leu Leu Ala Asp Met Asp Val Leu Leu Ser Phe Ala Asp Leu
            595                 600                 605

gct tcc tgc agt ccc gta cct tac gtc agg ccc tcg atc act tct gct      2114
Ala Ser Cys Ser Pro Val Pro Tyr Val Arg Pro Ser Ile Thr Ser Ala
        610                 615                 620

gat gaa ggt gac ata att ctg gaa gga agt cgg cat cca tgt gta gaa      2162
Asp Glu Gly Asp Ile Ile Leu Glu Gly Ser Arg His Pro Cys Val Glu
    625                 630                 635

gcc cag gat gac gtc aat ttc att tcc aac gat tgt caa ttg gtg cga      2210
Ala Gln Asp Asp Val Asn Phe Ile Ser Asn Asp Cys Gln Leu Val Arg
640                 645                 650                 655

ggc aag agc tgg ttt cag att atc aca gga ccc aat atg ggt gga aag      2258
Gly Lys Ser Trp Phe Gln Ile Ile Thr Gly Pro Asn Met Gly Gly Lys
                660                 665                 670

tcc acc ttc att aga cag gtt ggt gtg aac gtg tta atg gct caa gtt      2306
Ser Thr Phe Ile Arg Gln Val Gly Val Asn Val Leu Met Ala Gln Val

            675                 680                 685

gga tgc ttt gta cca tgc gac cga gca gag atc agt gtg cgt gat tgc 2354
Gly Cys Phe Val Pro Cys Asp Arg Ala Glu Ile Ser Val Arg Asp Cys
        690                 695                 700

atc ttt gcg cgt gtt ggt gct gga gat tgc cag cta cgc gga gtg cca 2402
Ile Phe Ala Arg Val Gly Ala Gly Asp Cys Gln Leu Arg Gly Val Pro
    705                 710                 715

aca ttt atg gct gaa atg ctg gaa aca gcc agc att att aag tct gct 2450
Thr Phe Met Ala Glu Met Leu Glu Thr Ala Ser Ile Ile Lys Ser Ala
720                 725                 730                 735

aca gag cgt tct ttg atc att att gat gaa ctt ggt cgg ggt act tca 2498
Thr Glu Arg Ser Leu Ile Ile Ile Asp Glu Leu Gly Arg Gly Thr Ser
                740                 745                 750

act tac gat gga ttt ggt ttg gcg tgg gca ata tgt gag tac tta gtg 2546
Thr Tyr Asp Gly Phe Gly Leu Ala Trp Ala Ile Cys Glu Tyr Leu Val
            755                 760                 765

gag gtc act cgt gca ccc act ctc ttt gca acc cac ttt cac gag cta 2594
Glu Val Thr Arg Ala Pro Thr Leu Phe Ala Thr His Phe His Glu Leu
        770                 775                 780

cca gct tta gaa cat tca aac tct cca cct gtc cat gga cct ccc aga 2642
Pro Ala Leu Glu His Ser Asn Ser Pro Pro Val His Gly Pro Pro Arg
    785                 790                 795

ggt cca ctt gta ggg att gcc aac tat cac gtt agt gct cat atc gac 2690
Gly Pro Leu Val Gly Ile Ala Asn Tyr His Val Ser Ala His Ile Asp
800                 805                 810                 815

aca aaa tca cag aaa ctt gcc atg ctc tac aag gtt gaa gaa gga cct 2738
Thr Lys Ser Gln Lys Leu Ala Met Leu Tyr Lys Val Glu Glu Gly Pro
                820                 825                 830

tgc gac caa agt ttt ggt atc cat gta gct gag ttt gcg cat ttt cct 2786
Cys Asp Gln Ser Phe Gly Ile His Val Ala Glu Phe Ala His Phe Pro
            835                 840                 845

gaa agt gtt gtt gaa ctc gct aag cag aaa gcg gca gaa tta gag gat 2834
Glu Ser Val Val Glu Leu Ala Lys Gln Lys Ala Ala Glu Leu Glu Asp
        850                 855                 860

ttc tct gat act ggg aag gat gtt tca ggg ggg ctt agt aag gat gag 2882
Phe Ser Asp Thr Gly Lys Asp Val Ser Gly Gly Leu Ser Lys Asp Glu
    865                 870                 875

gtt ggc act aag cga aag aga gta agt ggc cct gat gac aag gta tta 2930
Val Gly Thr Lys Arg Lys Arg Val Ser Gly Pro Asp Asp Lys Val Leu
880                 885                 890                 895

gga gct ggg cgg gtg cga agg ttc ctt caa gag atg gca gca ttg cct 2978
Gly Ala Gly Arg Val Arg Arg Phe Leu Gln Glu Met Ala Ala Leu Pro
                900                 905                 910

gta gac aaa atg agt cgt gag gag act tat gcc aaa tta aag gcc ttg 3026
Val Asp Lys Met Ser Arg Glu Glu Thr Tyr Ala Lys Leu Lys Ala Leu
            915                 920                 925

aaa agt gag ttt gac aga gac gcc gca aca aac tca tgg ctg caa cag 3074
Lys Ser Glu Phe Asp Arg Asp Ala Ala Thr Asn Ser Trp Leu Gln Gln
        930                 935                 940

ata gtt cat tga aaggttctct gaacgttatg tacatttgat ctagagacaa 3126
Ile Val His

    945

acatgtttta tttatgaata tatcttcctt gtgaggtgaa gtaaatgatg atgaaatgtg 3186

aagtaatttt tttgatccgt agctttgaca aaatacttat ctgtctggac gaaacgcagt 3246

cttcaagctg ttgtattgtt cgacttattg ataaagaggg aaactaatga ttcacacaaa 3306

aa 3308

<210> 2
<211> 946
<212> PRT
<213> Physcomitrella patens

<400> 2

Met Glu Lys Gly Asp Glu Pro Lys Leu Pro Glu Leu Lys Leu Asp Val
1               5                   10                  15

Lys Gln Ala Gln Gly Phe Ile Ser Phe Phe Arg Arg Leu Pro Glu Asn
            20                  25                  30

Glu Gln Ala Val Arg Phe Phe Asp Arg Arg Asp Tyr Phe Thr Ala His
        35                  40                  45

Gly Asp Asn Ala Thr Phe Ile Ala Ser Thr Tyr Tyr Arg Thr Leu Thr
    50                  55                  60

Val Leu Arg Gln Leu Gly Ser Gly Ala Asp Ser Leu Pro Gly Val Thr
65                  70                  75                  80

Ile Asn Lys Ser Met Phe Glu Thr Ile Val Arg Asp Leu Leu Leu Glu
                85                  90                  95

Arg Thr Asp Cys Thr Val Glu Leu Tyr Glu Gly Thr Gly Ser Lys Trp
            100                 105                 110

Gln Leu Val Arg Ser Gly Ser Pro Gly Lys Leu Gly Ser Phe Glu Glu
        115                 120                 125

Val Leu Phe Ala Ser Asn Glu Met Gln Glu Thr Pro Val Val Met Ala
    130                 135                 140

Val Asn Ile Thr Thr Arg Gln Ser Glu Arg Cys Val Gly Ile Ala Phe
145                 150                 155                 160

Val Asp Met Thr Lys Arg Gln Leu Gly Met Thr Glu Phe Leu Asp Asp
                165                 170                 175

Asp Leu Tyr Thr Ser Leu Glu Ser Ala Met Val Ala Leu Ser Cys Arg
            180                 185                 190

Glu Cys Ile Ile Pro Met Pro Thr Ala Ala Lys Ser Pro Asp Asp Arg
        195                 200                 205

Lys Leu Arg Asp Val Met Ala Arg Cys Asn Val Leu Val Thr Glu Lys
    210                 215                 220

Lys Lys Ser Asp Phe Arg Ser Arg Asp Val Glu Gln Asp Leu Gly Arg
225                 230                 235                 240

Leu Val Lys Gly Pro Ala Glu Gln His Lys Glu Leu Val Ala Ala Ser
                245                 250                 255

Asp Leu Ala Ala Ala Ala Leu Ala Ala Leu Leu Thr Tyr Thr Glu Leu
            260                 265                 270

Leu Ser Asp Asp Thr Asn Tyr Gly Lys Tyr Val Ile Gln Pro Tyr Ser
        275                 280                 285

Leu Asp Leu Tyr Met Arg Leu Asp Ala Ala Ala Leu Arg Ala Leu Asn
    290                 295                 300

Val Thr Glu Ser Lys Thr Asp Ser Asn Lys His Leu Ser Leu Phe Gly
305                 310                 315                 320

Leu Met Asn Arg Thr Cys Thr Gln Gly Met Gly Lys Arg Leu Leu Asn
                325                 330                 335

Arg Trp Leu Lys Gln Pro Leu Val Asp Val Val Glu Ile Thr Lys Arg
            340                 345                 350

Leu Asp Thr Val Gln Ala Phe Val Glu Asp Leu Glu Leu Arg Gln Asp
        355                 360                 365

Leu Arg Ser His Leu Arg Arg Met Pro Asp Ile Glu Arg Leu Val Ser
    370                 375                 380

Lys Leu Glu Lys Arg Lys Ala Gly Leu Gln Asp Val Val Arg Leu Tyr
385                 390                 395                 400

Gln Ala Ser Leu Arg Leu Pro Leu Ile Arg Thr Cys Leu Glu Lys Tyr
                405                 410                 415

Asp Gly Glu Phe Ser Lys Asp Leu Gln Glu Lys Tyr Gly Ala His Leu
            420                 425                 430

Asp Val Trp Thr Gln Pro Asn His Leu Gly Lys Phe Asp Gly Leu Val
        435                 440                 445

Glu Ala Ala Val Asp Leu Glu Gln Leu Gln Asn Gly Glu Tyr Ile Tyr
    450                 455                 460

Phe Ser Arg Leu Cys Gln Gln Ser Ser Arg Ala Gln Asn Trp Ala Arg
465                 470                 475                 480

Arg Cys Glu Arg Gln Ile Leu Lys Val His Gln Gln Ser Ala Asp Asp
                485                 490                 495

Leu Gly Leu Pro Ala Glu Lys Ser Leu Lys Leu Asp Lys Ser Thr Gln
            500                 505                 510

Tyr Gly His Val Phe Arg Ile Thr Lys Lys Glu Glu Pro Lys Val Arg
        515                 520                 525

Lys Lys Leu Asn Ser Gln Tyr Ile Thr Leu Glu Thr Arg Lys Asp Gly
    530                 535                 540

Ile Lys Phe Thr Asn Ala Lys Leu Arg Arg Leu Ser Glu Gln Tyr Thr
545                 550                 555                 560

Lys Leu Thr Glu Glu Tyr Ser Ser Ala Gln Arg Glu Leu Val Ala Lys
                565                 570                 575

Val Val Asp Val Ala Ala Thr Phe Val Glu Ile Phe Ser Gly Val Ala
            580                 585                 590

Val Leu Leu Ala Asp Met Asp Val Leu Leu Ser Phe Ala Asp Leu Ala
        595                 600                 605

Ser Cys Ser Pro Val Pro Tyr Val Arg Pro Ser Ile Thr Ser Ala Asp
    610                 615                 620

Glu Gly Asp Ile Ile Leu Glu Gly Ser Arg His Pro Cys Val Glu Ala
625                 630                 635                 640

Gln Asp Asp Val Asn Phe Ile Ser Asn Asp Cys Gln Leu Val Arg Gly
                645                 650                 655

Lys Ser Trp Phe Gln Ile Ile Thr Gly Pro Asn Met Gly Gly Lys Ser
            660                 665                 670

Thr Phe Ile Arg Gln Val Gly Val Asn Val Leu Met Ala Gln Val Gly
        675                 680                 685

Cys Phe Val Pro Cys Asp Arg Ala Glu Ile Ser Val Arg Asp Cys Ile
    690                 695                 700

Phe Ala Arg Val Gly Ala Gly Asp Cys Gln Leu Arg Gly Val Pro Thr
705                 710                 715                 720

Phe Met Ala Glu Met Leu Glu Thr Ala Ser Ile Ile Lys Ser Ala Thr
                725                 730                 735

Glu Arg Ser Leu Ile Ile Ile Asp Glu Leu Gly Arg Gly Thr Ser Thr
            740                 745                 750

Tyr Asp Gly Phe Gly Leu Ala Trp Ala Ile Cys Glu Tyr Leu Val Glu
        755                 760                 765

Val Thr Arg Ala Pro Thr Leu Phe Ala Thr His Phe His Glu Leu Pro
    770                 775                 780

Ala Leu Glu His Ser Asn Ser Pro Pro Val His Gly Pro Pro Arg Gly
785                 790                 795                 800

Pro Leu Val Gly Ile Ala Asn Tyr His Val Ser Ala His Ile Asp Thr
                805                 810                 815

Lys Ser Gln Lys Leu Ala Met Leu Tyr Lys Val Glu Glu Gly Pro Cys
            820                 825                 830

Asp Gln Ser Phe Gly Ile His Val Ala Glu Phe Ala His Phe Pro Glu
        835                 840                 845

Ser Val Val Glu Leu Ala Lys Gln Lys Ala Ala Glu Leu Glu Asp Phe
    850                 855                 860

Ser Asp Thr Gly Lys Asp Val Ser Gly Gly Leu Ser Lys Asp Glu Val
865                 870                 875                 880

Gly Thr Lys Arg Lys Arg Val Ser Gly Pro Asp Asp Lys Val Leu Gly
                885                 890                 895

Ala Gly Arg Val Arg Arg Phe Leu Gln Glu Met Ala Ala Leu Pro Val
            900                 905                 910

Asp Lys Met Ser Arg Glu Glu Thr Tyr Ala Lys Leu Lys Ala Leu Lys
        915                 920                 925

Ser Glu Phe Asp Arg Asp Ala Ala Thr Asn Ser Trp Leu Gln Gln Ile
    930                 935                 940

Val His
945

<210>  3
<211>  6681
<212>  DNA
<213>  Physcomitrella patens

<400>  3
agtcgaattt gcgaagccgt cgactggagc gagattgtgt tagcgccgct ctggtaatct      60

agtcagaagg gttcgtgtta tggcttgatt cttgaattgg cgcgatcatt aggtttttag     120

cgtcgaggac gttggtgtga tccatgtcgg aggtgcatgc aggtcagcct tcttcatact     180

acacctcggt gagttcggga tttgggagcg caatttcgta ggagcttgaa gtattttcgc     240

ttttcgagtg ctttgagcac cgaggcgttg cgtttttttg aagatttgaa tacctacagc 300
atagttagtg ttggatttgg caatccgatt gtgggatgga cccagatagt atggagaagg 360
gggatgagcc gaagctgccc gaattgaagc taggtacatg aatttttcct cgcattggta 420
atctttgaca ctgaatgagt gacggtgtag ttttcggacc ggaacggtgc ttgaaaggaa 480
gatatctaag aagagaaggg gtttttcagg ttttggacgt agcagctagt tacatttgaa 540
aattggcgag gcaattcgtc caatgcctgt gtctaagatt accggaagtg tgaggaaact 600
tcaatcactt gccaacagga ccgtggagag tagatctaag tctaacgact gtgccctatc 660
ttgtggatgt cgtatgcaga tgttaagcaa gcacaggggt tcatttcttt cttccgcagg 720
cttcccgagg tttgtatttg ttctacccga tatctcatga agaaattcgt tggtagttcc 780
ctcatgttgt gggacaaggc atcctatctg cgacatctta aaccccttca gtatgcagct 840
cgaagtgttc aaagagcttg cgtctgcgta gaacataaag agactacctc atatcacaca 900
cgagatctct gtactccttt cgcagaaaaa agaacacaat gcgtaagatt tgttcgtcat 960
ttcaatgcct tacttagcaa ggcatttcag tgatccgaat ggcttgatat gttggagtta 1020
tgtgtcttac agctggattg atgtcctgct tgatgcttat gcagaacgag caagctgtgc 1080
gcttctttga ccgcaggtga aactagaatt agcccttgct agcttttgtt atatgcccaa 1140
tatttgttct tgcgtgccgg agcttctcat agtgggctgt taaaaaaatt ctttaaagag 1200
cgtagaaata cgtgtttcca ctgaattaat tattctatca ctgcgtctat cttgagtatt 1260
tttgtaggtc tcttatgggt gtcactaagt ccttaatatt atccgtggtt atccgatgta 1320
ttctgtacca caggattatt tcactgctca tggtgataat gcaaccttca ttgccagtac 1380
gtactatagg acgctcacag ttctcaggca gctggggagc ggggctgatt ctctccctgg 1440
tgtgaccatc aacaaaagca tgtttgagac tattgtgagg gatctcttgc tagaaagaac 1500
tgattgcact gttgaactct atgagggcac cggatctaag tggcaacttg ttaggagcgg 1560
gtctcctggc aagcttggca gctttgagga ggtcctgttc gcaagcaatg agatgcaggt 1620
atagtcgaaa aaatttggaa tgtctggaat gtctaccgaa gctatggaga aactggacac 1680
tgttaggatg agattgtctt tgaaacgcat gcatttggtt actctgtgaa gcattcgagt 1740
taattttcca ttctgaagtt gtgttatttc aaattatctg gttcactgta cttacaacct 1800
ccctcttcaa tactcaggaa actcctgtag tcatggctgt aaatattact acacgtcaaa 1860
gcgagcggtg tgttgggata gcattcgtgg atatgacgaa gaggcagctt ggaatgacag 1920
agttcttaga cgatgaccta tacaccagtt tggagtccgc catggtagct ttaagctgtc 1980
gtgaatgtat catacccatg ccaaccgctg ccaagtcccc tgatgatcgt aaacttcgtg 2040
atgtaatggc acgctgcaat gttttggtta ctgagaagaa gaaatctgat tttaggtacg 2100
ctgtttttct gcagtcaacg gccgcactgg catccataaa caatgaccat tattatcgca 2160
gaaatgcttg tgacaatggt ttgtgtgcgt cggctggtta tacttcagaa ccctctccga 2220
cctgattctg ggcttgtgca ggtcgcgaga tgtagaacaa gatttaggcc gattagtcaa 2280

aggtcctgct gagcaacata aggaactggt ggcagcatct gatttagcgg ctgctgctct 2340
tgccgcgctt ctgacatata ctgaactcct ctctgatgac acaaattatg gaaaatacgt 2400
cattcagccc tacagtctgg acctctacat gcgattggat gcagcagctt tacgagctct 2460
caatgtcact gagagcaaaa cggattctaa caagcatctt agtctgtttg gtttgatgaa 2520
ccggacttgc acccaaggta tgggaaaaag attgctgaac cggtggttga aacaacccct 2580
agtggatgtt gttgagataa ccaaaaggct ggatacagtg caagcgtttg tggaggatct 2640
agaattaagg caggacttgc ggtctcacct acggcgaatg cctgacattg agcgcttggt 2700
tagcaagctt gagaagcgca aagcgggatt gcaagatgtt gtgagattgt atcaggtgaa 2760
tcgcatgtga cgtgttattg atctcttgtt attttacaat taagcctaca aaggagtaaa 2820
caagatgtca tggcttattg tgtggttttc atccatcttt atggtagata tgctctccgc 2880
tatatggtta atcacacgat ttctattgaa attttgatgc tttaggttcc ttgtgtccca 2940
agtcgtgggt ggaattacaa ggtagtcgcg aacttcagaa tcataattgt tctcaaattt 3000
gtcatcagat ctcagaggta ttttaaagcg agatattatt gtttgcaggc cagtttgagg 3060
ctgcctttga ttcgaacctg cctggagaag tatgacggag agttttccaa ggatttgcaa 3120
gagaaatacg gcgcccatct tgatgtatgg acacagccaa atcatctagg aaaatttgat 3180
ggattagtcg aggcagctgt cgatctcgaa cagctccaga atggagagta tatatatttc 3240
agcagattat gccaacagtc ttcaagagct caaaactggg cgcgacgatg tgaaaggcag 3300
atacttaaag ttcaccagca gtcagcagat gacctggggc ttcctgcgga aaagtcattg 3360
aagttggata agagtacaca gtatggacat gtttttagga ttactaagaa ggaagagccg 3420
aaggttagga aaaaattgaa tagtcaatac ataaccttgg aaactaggaa agacggcata 3480
aagttcacga atgccaagct gcgaaggttg agtgaacagt acacaaagct cacagaggaa 3540
tactcgagtg cacagcgaga gcttgttgca aaggttgtgg atgttgcagc aacatttgtt 3600
gaggcaagtt tctctactgt ttacattagt cttacttgta tttcataatg tatgttttct 3660
tcgataccaa ttcccctaca tagtcttccc aagcactcga cgccttaatc atgcagatat 3720
tctttcctgt ttttgagtgt gtcatattaa acatgcatct ctgtccattc ccttccagat 3780
tttctcagga gttgcagttc ttctggctga tatggatgta cttcttagct ttgctgactt 3840
agcttcctgc agtcccgtac cttacgtcag gccctcaatc acttctgctg taagctactc 3900
ttgacactga aaccacttaa tgtgtatata gcttattgtg ctggaaacca cgccaaagtc 3960
aagattgtgg tttttttctg tgaatgttag tccgtattca gtccttaaag ttactatttg 4020
taaacaaggc attctttcca acttgagact atgcagatga cgtgggccat tttgatttca 4080
ggatgaaggt gacataattc tggaaggaag tcggcatcca tgtgtagaag cccaggatga 4140
cgtcaatttc atttccaacg attgtcgatt ggtatagtgg agtagaaatc acaatcttct 4200
ctttggcttt tgaattcctt tacctgttta gcttagtttc agtgttgaat ttgaattgtt 4260
gaacttctag gtttatcgtc tattcttcag tttactaact actcttataa acagaacttt 4320

ggagaaataa ttttgtcact tgtttctctc aggtgcgagg caagagctgg tttcagatta 4380
tcacaggacc caatatgggt ggaaagtcca ccttcattag acaggtgaag tttatcacct 4440
ttttttctac ttgtttcctt cgaaaaagtg gatgggagga tagaaaccgc aattgctgct 4500
gcaaatggac ctaagccctc attaagattt gatgttccgt ggaactaatc actgacacgg 4560
ttagtgattg tccggatgga cttgttgttt ggctgtgtaa tttcaccgtg tggaagtact 4620
tgcctgcatc tagcagattt atttggtcgt gacaaggtca tgatatatcg tcattcagct 4680
gggactgact tatggcacac tggccttcgt tgtcaatttt taagtagcat tcttgatgtt 4740
gcttgatgtc tcatcttcag gttggtgtga acgtgttaat ggctcaagtt ggatgctttg 4800
taccatgcga ccgagcagag atcagtgtgc gtgattgcat ctttgcgcgt gttggtgctg 4860
gagattgcca ggtactaata tacgcatgct ctcttagtgc gtatcagcgc cactctgcga 4920
aacttctggg tgtttcgtct atttagaact gcgccagatt tatgttgtta acaatcagga 4980
tgtgaaattg ctgtttacgt tttatacctc tattcttttg cagctacgcg gagtgtcaac 5040
atttatggct gaaatgctgg aaacagccag cattattaag tctgctacag agcgttcttt 5100
gatcattatt gatgaacttg gtcggggtac ttcaacttac gatggatttg gttagttctc 5160
tttataacct ttcatggaaa gaggttgacc aaatactttt ccttcatgtg gttaagctgt 5220
aggctaatgg agcttgggac ttgcgagcag ggtgagggtt cactgttatc ttttgaaatc 5280
atgttagtca ttagagtcgt ctgttttgtt ctgttcttgt cgtgaagtta ccgagttcag 5340
atgcaatgtc ttcaggttaa ctccattttt tttattatga cacattcaaa gagcatttat 5400
atgtcgatac tatttgtgct aaaccatctg aaatacaggt ttggcgtggg caatatgtga 5460
gtacttagtg gaggtcactc gtgcacccac tctctttgca acccactttc acgagctaac 5520
agctttagaa cattcaaact ctccacctgt ccatggacct cccagaggtc cacttgtagg 5580
gattgccaac tatcacgtta gtgctcatat cgacacaaaa tcacagaaac ttgccatgct 5640
ctacaaggta attttttttt taatgaatat atatatatat atatatatat attaatcgtg 5700
agtgtagtac tgtaggaagt gtgcagcaat cagaagtctt gcaaatgaat agcttcgtta 5760
gctaattaag cgaatgtatt aagtggcgga aatactttca gacctttctg ccctgtcatc 5820
cacaaaaatt gcgttggggt ttctcatcta tatcgtgttc gattgtacag gttgaagagg 5880
gaccttgcga ccaaagtttt ggtatccatg tagctgagtt tgcgcatttt cctgaaagtg 5940
ttgttgaact cgctaagcag aaagcggcag aattagagga tttctctgat actgggaagg 6000
atgtttcagg ggggcttagt aaggatgagg tgcgatttaa tctgttctgt tgtgttattc 6060
atattccaat aggtgtatgt cttgtaaatt gtgatcactg acagagagtt cttgttctct 6120
atcttttgac atattcagac attgagtgat acttatatta acaagcgttt gttttagtga 6180
tcattaacaa agataaactg cattcgccgt ttgatgctcg tttgagttct tacgcgttga 6240
ttttgatatt taataggttg gcactaagcg aaagagagta agtggccctg atgacaaggt 6300
attaggagct gggcgggtgc gaaggttcct tcaagagatg gcagcattgc ctgtagacaa 6360

aatgagtcgt gaggagactt atgccaaatt aaaggccttg aaaagtgagt ttgacagaga 6420

cgccgcaaca aactcatggc tgcaacagat agttcattga aaggttctct gaacgttatg 6480

tacatttgat ctagagacaa acatgtttta tttatgaata tatcttcctt gtgaggtgaa 6540

gtaaatgatg atgaaatgtg aagtaatttt tttgatccgt agctttgaca aaatacttat 6600

ctgtctggac gaaacgcagt cttcaagctg ttgtattgtt cgacttatga taaagaggga 6660

aactaatgat tcacacaaaa a 6681

<210> 4
<211> 22
<212> DNA
<213> artificial sequence

<220>
<223> PpMSH2#4

<400> 4
caatgtcact gagagcaaaa cg 22

<210> 5
<211> 22
<212> DNA
<213> artificial sequence

<220>
<223> primer pPMSH2#5

<400> 5
cgaccgagca gagatcagtg tg 22

<210> 6
<211> 27
<212> DNA
<213> artificial sequence

<220>
<223> Primer CaMV35S-Pro

<400> 6
tatttttgga gtagacaagc gtgtcgt 27

<210> 7
<211> 24
<212> DNA
<213> artificial sequence

<220>
<223> primer CaMV-Ter

<400> 7
ccgcttcctc gtgctttacg gtat 24

<210> 8
<211> 19
<212> DNA
<213> artificial sequence

<220>
<223> primer PpMSH2#14

<400>  8
cgactggagc gagattgtg                                                    19


<210>  9
<211>  19
<212>  DNA
<213>  artificial sequence

<220>
<223>  primer PpMSH2#15

<400>  9
caacagcttg aagactgcg                                                    19

## Description

The invention relates to a method for producing new transgenic or hybrid plants by homeologous recombination, and to its application for plant genetic engineering and plant breeding.

The methods of genetic modification rely on the ability of virtually every cell type to exchange DNA with a high degree of nucleotide sequence similarity by a process which is called homologous recombination. This process, which is observed during mitosis and meiosis, provides a basis for performing specific targeted alterations in the genome of an organism. The method may comprise: (i) either the introduction in said organism or cells thereof, of extrachromosomal DNA homologous to the target sequence and the selection in the transformants of those which have integrated the extrachromosomal DNA by homologous recombination at the target locus (genetic transformation), or (ii) the crossing of an organism with an organism from an other species expressing a trait of interest encoded by a donor gene, and the selection in the progeny, of those which have integrated the donor gene by inter-chromosomal homologous recombination at the target locus (plant or animal breeding). Targeted homologous recombination has been proposed to create transgenic organisms such as plants and animals. Homologous recombination permits the targeted change to be incorporated into homologous sites in chromosomes of the organism so that the change may be passed on to the organism's progeny.

However, the efficiency of homologous recombination in both prokaryotes and eukaryotes strongly depends on complete sequence identity of exchanging DNA strands. Thus, sequence dissimilarities as small as 0.5 % can already strongly impede homologous recombination. Therefore, the limits within which homologous recombination occurs, define a barrier beyond which genome modification at a target locus cannot be performed, either by transforming an organism or cells thereof or by crossing organisms from different species.

Mismatch repair (MMR) systems are highly conserved evolutionarily and have important function in maintaining eukaryotic genome stability (Modrich and Lahue, Annu. Rev. Biochem., 1996, 65: 101-133). The MMR system is known better for its role in the post-replicative repair of DNA polymerization errors, resulting from nucleotide misincorporation and polymerase slippage, which is crucial for keeping mutation rates at an acceptably low level (Glickman and Radman, Proc. Natl. Acad. Sci. USA, 1980, 77: 1063-1067; Claverys and Lacks, Microbiol. Rev., 1986, 50: 133-165). In addition MMR proteins also recognize mismatches in heteroduplex recombination intermediates. This anti-recombination activity of MMR proteins promotes genome stability by inhibiting interactions between diverged sequences present in a single genome or derived from different organisms (Rayssiguier et al., Nature, 1989, 342: 396-401). The MMR system also plays a role in some types of nucleotide excision repair responsible for repair of physical/chemical damage to DNA, and finally participates in a cell-cycle check-point control system by recognizing certain types of DNA damage and triggering cell-cycle arrest or other responses to DNA damage.

MMR systems contain proteins homologous to the well-characterized methyl-directed DNA repair system of *Escherichia coli,* proteins MutS and MutL [Mut for mutator phenotype because they have been isolated from mutant strains with elevated frequencies of spontaneous mutations; Kolodner et al., Gene Dev., 1996; 10: 1433-1442; Modrich and Lahue, 1996]. These key proteins in MMR are highly conserved from bacteria to mammals. In eukaryotes, there are multiple homologs of the key bacterial MutS and MutL MMR proteins, and these homologs form heterodimers that have discrete roles in MMR-related processes (Harfe and Jinks-Robertson, Annu. Rev. Genet., 2000: 34, 359-399). Six different genes homologous to *mutS* have been isolated in yeast *(ymsh),* and their homologs have been found in mouse *(mmsh),* human (*hmsh*) and a number of plant species *[msh2* in Tobacco *(Nicotiana tabacum;* International PCT Application WO 03/054217), maize *(Zea mays;* Genbank accession number AJ238785) and *Arabidopsis thaliana* (Genbank accession number AF026549); *msh3* and *msh6* in *Arabidopsis thaliana* (United States Patent n° 6,734,019)]. Encoded proteins yMSH2, yMSH3 and yMSH6 appear to be the main MutS homologs involved in MMR during mitosis and meiosis in yeast, where the complementary proteins MSH3 and MSH6 alternatively associates with MSH2 to recognize different mismatch substrates (Masischky et al., Genes Development, 1996, 10: 407-420). Similar protein interactions have been demonstrated for the human homologs hMSH2, hMSH3 and hMSH6 (Acharya et al., P.N.A.S., 1996, 93: 13629-13634). The current model for nuclear DNA MMR in eukaryotes proposes that heterodimers can recognize and stimulate repair of single base-pair mismatch and small insertion/deletion loops (Harfe and Jinks-Robertson, 2000). MSH2 is present in all heterodimers, thus is crucial for the repair of all mismatched lesions, whereas other MSH modulate the function of MSH2 by providing specificity for different lesion types.

However, whereas in several bacterial species, yeast and mammalian cells it has been unequivocally shown that the DNA mismatch repair system is responsible for suppressing recombination between homologous but nonidentical sequences (homeologous recombination), in plants, partial information only, is available on MMR system and the role of the MMR genes in the mechanism of homologous recombination itself has not been established.

In bacterial MMR mutants *(mutS* and *mutL),* interspecies recombination occurs efficiently between *Escherichia coli* and *Salmonella typhimurium,* which are - 20% divergent in DNA sequence (Rayssiguier et al., 1989).

In yeast and mammals, *msh2* mutation phenotypes are: (1) a microsatellite instability, (2) a mutator phenotype characterized by a high spontaneous mutation rate, (3) an elevated recombination between sequences diverging in the range of 0.1% to 1.5% and (4) tolerance to some type of chemical / physical treatments damaging DNA (Negritto et al., Molecular and Cellular Biology, 1997, 17: 278-286; de Wind et al., Cell, 1995, 82: 321-330; Abuin et al., Mol. Cell. Biol., 2000, 20: 149-157; Elliot and Jasin, Mol. Cell. Biol., 2001, 21: 2671-2682). In *Caenorhabditis elegans,* loss of MSH2 is associated with microsatellite instability, mutator phenotype and a reduced fertility (Degtyareva et al., Proc. Natl. Acad. Sci., USA, 2002, 99: 2158-2163).

In plants, Hoffman and coworkers (Genes and development, 2004, 18: 2676-2685) have shown in *Arabidopsis thaliana* a rapid accumulation of a wide variety of mutations during seed-to-seed propagation of *Atmsh2* defective lines and microsatellites instability, but the role of *msh2* and/or other MMR genes in the mechanism of homologous recombination itself has not been established.

Based on the findings in bacterial, yeast and mammalian cells only, a new method based on the genetic and/or functional inactivation of the MMR system has been proposed for modifying bacterial, yeast and higher eukaryotes genome by homeologous recombination with DNA targeting constructs which substantially differ from the target locus in the region where recombination takes place. The genetic inactivation of the MMR system comprises disrupting both copies of one gene essential for DNA mismatch repair, for example the *msh2* gene (International PCT Applications: WO 90/07576 (bacteria), WO 97/37011 (yeast), WO 97/05268 (mammal), WO 03/054217 (plant)), or the *msh3* and/or *msh6* genes (United States Patent n° 6,734,019). The functional inactivation of the MMR system comprises (i) introducing antisense oligodeoxynucleotides or antisense RNA constructs, (ii) expressing a dominant negatively acting version of a gene involved in DNA mismatch repair, for example expressing the prokaryotic MMR proteins (MutS) in eukaryotic cells, and (iii) saturating the DNA mismatch repair system by the introduction into the cell, of DNA molecules carrying one or more mis-or unpaired bases. (International PCT Applications: WO 90/07576, WO 97/37011, WO 97/05268, WO 03/104451 and WO 03/054217; United States Patent n° 6,734,019).

This method may be used for engineering transgenic organisms, for modifying eukaryotic species of which inbred strains are not easily available, and also for repairing somatic mutations (gene therapy).

However, this method was not proven successful yet in higher eukaryotic organisms since MMR gene disruption is associated with a mutator phenotype and a reduced fertility, as demonstrated with *msh2* mutants in mice (de Wind *et al.,* 1995). In addition, the efficacy of the functional inactivation of the MMR system has not been established in higher eukaryotic cells.

To determine the role of MSH2 in plant DNA metabolism, the inventors have cloned the *msh2* gene in *Physcomitrella patens,* they have generated *Physcomitrella patens msh2* disruptants, and they have demonstrated MSH2 roles, by using the well-known adenine phosphoribosyltransferase (APT) gene of *P. patens* as reporter gene (Houba-Herin et al., in: Peth J.C., Latché A., Bouzayen M (Eds), Plant Sciences, 1997*,* 3e Colloque Général de la Société Française de Physiologie vigitale, SFPV-INRA, Toulouse, 1997, pp, 22-23 *;* Schaefer D.G., Curr. Opin.Plant Biol., 2001, 4: 143-150). Indeed unique in the plant kingdom, the moss *Physcomitrella patens* allows gene targeting via homologous recombination (Schaefer DG, Annu Rev. Plant Biol., 2002, 53: 477-501). The PpMSH2 cDNA has been isolated (Brun et al., Biochimie, 2001, 83, 1003-1008); it encodes a MSH2 protein corresponding to the sequence SEQ ID NO: 2 in the attached sequence listing. Thus, gene knock-out and allele replacement approaches are feasible in this organism. It also makes the moss a convenient model to study the regulation of homologous recombination in plants.

The inventors have shown that in plants, MSH2 is involved in genome stability and its absence leads to a mutator phenotype. Moreover, they have demonstrated that MSH2 constitute a barrier to recombination by preventing homologous recombination between diverged DNA sequences. They have also demonstrated that the lack of MSH2 allows targeting to a locus with sequences which have at least 3 % and up to 10 % differences, as efficiently as with perfectly identical sequences. This is the first demonstration of gene targeting with homeologous sequences in plants. In addition, this is also the first demonstration of gene targeting in higher eukaryotic organisms, with sequences which have such a degree of divergence ; indeed, it has only been shown so far, that gene targeting in mouse cells can be performed with sequences having up to 1.5 % differences (De Wind et al., 1995; International PCT Application WO 97/05268).

This finding provides a route for the production of new transgenic and new hybrid plants in which alleles or variants of genes encoding desirable production or quality traits could be inserted at a desired locus by homeologous recombination. These genes include for example, alleles of genes present in other species but which where unusable to breeders due to genetic isolation, or variants which have been produced by genetic engineering but which where difficult to insert by homologous recombination due to their divergence with the wild type gene.

Consequently, the invention provides a method for producing a hybrid or transgenic plant, or cell thereof, comprising at least the steps of:
a) inactivating transitorily MSH2 in a plant or cell thereof,
b) introducing in said plant or cell thereof, a targeting sequence having from 0.1 % to 10 % differences with a target sequence in the genome of said plant or cell thereof, under conditions allowing homeologous recombination to occur between said target and targeting sequences, and
c) identifying the hybrid or transgenic plant or cell thereof, in which genomic integration of said targeting sequence as occurred through homeologous recombination with said target sequence.

It is referred to here as homologous recombination if donor and recipient sequences are identical or nearly identical (at least 99.9 % sequence identity), and as homeologous recombination if donor and recipient sequences are not identical but are similar (less than 99.9 % identity).

The MSH2 activity refers to the inhibition of homeologous recombination (anti-recombination activity). This activity may be evaluated by measuring gene targeting efficiency at the locus of a reporter gene (target sequence) with homologous sequence and homeologous sequence, in an appropriate system, for example in *Physcomitrella patens.* For example, protoplasts are transformed with vectors containing a positively selectable marker gene flanked by sequences of at least 50 nucleotides in length, preferably at least 200 nucleotides, said sequences being either homologous sequences or homeologous sequences having preferably from 3 % to 10 % differences with said target sequence, and transformants are selected. The gene targeting events in the transformants are detected by an appropriate assay; for example if disruption of the reporter gene confers resistance to a drug, the gene targeting events are detected by counting the number of resistant colonies in the presence of the drug. Gene targeting frequency is calculated by the ratio of mutants at the target locus to the number of stable transformants. In the presence of MSH2, gene targeting with homeologous sequence is very low as compared with homologous sequence. The inactivation of MSH2 is detected by an increase of gene targeting efficiency with homeologous sequence, to a level comparable to that observed with the homologous sequence.

The inactivation of MSH2 refers to the inhibition of MSH2 protein anti-recombination activity or *msh2* gene expression.

The inhibition of *msh2* gene expression comprises the functional and the genetic inactivation of the *msh2* gene, said genetic inactivation being partial or total.

The targeting sequence having from 0.1 % to 10 % differences with said target sequence, refers to a chromosomal or extrachromosomal nucleic acid molecule presenting 90 % to 99.9 % identity with said target sequence.

The percentage of sequence identity is determined by comparing optimally aligned target and targeting sequence over a comparison window, wherein the position of the polynucleotide sequence in the comparison window may comprise additions or deletions (i.e gaps) as compared to the reference sequence (which does not comprise additions or deletions) for optimal alignment of the two sequences.

The percentage is calculated by determining the number of positions at which the identical nucleic acid base occurs in both sequences to yield the number of matched positions, dividing the number of matched positions by the total number of positions in the window of comparison, and multiplying the result by 100 to yield the percentage of sequence identity.

This percentage may be determined by using one of the well-known alignment programs, using standard parameters.

Depending on the structure of the targeting sequence, the homeologous recombination may result in a deletion, insertion or substitution, at the target locus. For example, genomic sequences can be replaced by small oligonucleotides carrying one or more base pair alterations or by large chromosomal sequences derived from other species. The method can also be used to generate large deletions by intra-or extrachromosomal homeologous recombination between repeated but diverged sequences.

The targeting sequence according to the present invention represents an allelic locus, a non-allelic gene, a genetically engineered variant of a gene, or fragments thereof, which constitute or contribute to desired quantitative trait (growth performance, yield,) or qualitative trait [composition of leaves and seed storage products (oil, starch, protein, lignin content); prolonged flowering; altered morphology; pathogen resistance; tolerance to improved performance under environmental stresses of various kinds].

Examples of plants of the present invention include, but are not limited to angiosperms (crops, vegetables, ornamentals, conifers) and musci (mosses). Preferred crops include: corn, rice, rye, oats, barley, alfalfa, sunflower, soybean, cotton, safflower, peanut, sorghum, wheat, millet and tobacco.

The method of the invention allows the production of modified (transgenic or hybrid) plants by manipulating the genome of a plant, or derived tissues or cells. Modified tissues or cells may also be produced with the method of the invention, by manipulating the genome of plant tissues or cells thereof, or by isolating said tissues or cells from a modified plant.

In the present invention, "introducing a nucleic acid construct in a plant or cell thereof'', refers to presenting to a plant cell, regenerating tissue or whole plant, the nucleic acid construct in such a manner that the construct gains access to the interior of a cell of the plant. It includes transient transformation, wherein the nucleic acid construct does not integrate into the genome of the plant, and stable transformation, wherein the nucleic acid construct integrates into the genome of the plant and is capable of being inherited by progeny thereof.

According to a first advantageous embodiment of said method, the transitory inactivation of MSH2 comprises the introduction of an MSH2 inhibitor in said plant or cell thereof.

The term "MSH2 inhibitor" refers to any molecule which is able to inhibit the anti-recombination activity of MSH2, in a plant or cell therof and thereby increases homeologous recombination in said plant or cell, in which it is introduced. The inhibition may be evaluated by measuring gene targeting frequency with homologous and homeologous sequence, as described above.

The term "introduction" include: (i) the delivery of an exogenous molecule, in the form of the inhibitor itself or a polynucleotide construct encoding said inhibitor, for example a recombinant plasmid (transient transformation)), and (ii) the induction of the expression of the inhibitor from a polynucleotide construct encoding said inhibitor, said polynucleotide which is integrated in the genome of said plant or cell thereof being operably linked to an inducible promoter (stable transformation).

The MSH2 inhibitors include drugs such as cadmium (CdCl₂) and iododeoxyuridine (IdUrd) and dominant negative mutants of MSH2. The drugs are preferably used in the range of 1 to 50 µM.

The term "dominant negative mutants" refers to variants whose expression reduces the anti-recombination activity of MSH2 in a plant or cell. Such variants may be obtained by: (i) deletion, substitution or insertion of one or more amino acids in the MSH2 sequence, and (ii) selection of the mutants which are able to increase homeologous recombination in a plant or cell thereof, by measuring gene targeting efficiency at the locus of a target gene, as described above. Example of such mutants includes competitive inhibitors of MSH2 binding to its partners (MSH3 and or MSH6).

Preferred variants include at least one mutation selected in the group consisting of: S493P, K542E, G670D, K671R and S672P, wherein the positions are indicated by reference to *P. patens* MSH2 amino acid sequence SEQ ID NO: 2, which is encoded by the cDNA sequence SEQ ID NO: 1. Knowing the mutations in *P*. *patens* MSH2 sequence, one skilled in the art will find easily the corresponding mutations in the MSH2 sequence from other plants.

According to a second advantageous embodiment of said method, the transitory inactivation of MSH2 comprises the transitory inhibition of *msh2* gene expression in said plant or cell thereof.

When said inhibition is a functional inactivation of the *msh2* gene, it is preferably mediated by a polynucleotide encoding a sense, antisense, ribozyme, or RNAi molecule, which is similar (sense molecule) or complementary (antisense, ribozyme, RNAi) to the *msh2* transcripts, so as to decrease the level of MSH2 protein which is produced in the plant or cells thereof.

Said polynucleotide is transitorily expressed, either by transient transformation of said plant or cells thereof, for example with an expression plasmid comprising said polynucleotide, or by inducing the expression of said polynucleotide in stably transformed plant or cells, wherein said polynucleotide is operably linked to an inducible promoter.

Preferred RNAi molecule include RNAi targeting a sequence of at least 20 consecutive nucleotides situated between positions 950 and 1430 of MSH2 cDNA by reference to *P. patens* cDNA sequence SEQ ID NO: 1, preferably said RNAi targets the sequence from positions 950 to 1430. More preferably, said RNAi is a hairpin RNAi.

Knowing the positions of the target sequence in MSH2 cDNA, one skilled in the art will find easily the corresponding positions in the MSH2 cDNA sequence from other plants.

When said inhibition is a partial or total inactivation of the *msh2* gene, step a) may be performed with a *msh2* knock-out mutant, or a *msh2* variant (natural or recombinant) carrying an *msh2* allele which is : (i) functional during mitosis but not during meiosis (production of hybrid plants), or (ii) under the control of an inducible promoter which can be switch off transitorily (repressible promoter), to allow homeologous recombination to occur in step b) (production of transgenic plants). Examples of variants which are functional during mitosis but not during meiosis include those wherein the *msh2* gene is under the control of the CaMV 35S promoter. Examples of inducible promoter which can be switch off transitorily in plant cells are known in the art and any of these repressible promoters, such as a tetracycline repressible promoter, can be used in the present invention.

Furthermore, when *msh2* is totally inactivated in step a) (knock-out or disruption mutant), said method comprises an additional step d) of recovering MSH2 activity in said plant or cell thereof. Said recovery may be performed by stable transformation of the hybrid or transgenic plants obtained in c) with a polynucleotide encoding MSH2, operably linked to a promoter which is functional in plants and isolating the transformants expressing MSH2 (functional integration of the polynucleotide), or by crossing said hybrid or transgenic plants obtained in c), with a wild-type plant from the same species as the plant in step a) (recipient plant or recipient species), and isolating the recombinants carrying a (wild-type) *msh2* allele.

According to a third advantageous embodiment of the method of the invention, it is a method for producing a transgenic plant or cell thereof, wherein said targeting sequence in step b) is an extrachromosomal nucleic acid molecule.

Said method allows the production of new transgenic plants or cells thereof comprising variants of genes encoding desirable production or quality traits which where difficult to insert by homologous recombination due to their divergence with the wild type gene, but could be inserted at a desired locus by homeologous recombination.

The extrachromosomal nucleic acid molecule may be comprised of deoxyribonucleotides, ribonucleotides, and combination thereof. Such deoxyribonucleotides and ribonucleotides include, but are not limited to, naturally occurring and synthetic form, and derivatives thereof. The extrachromosomal nucleic acid molecule is for example a DNA molecule which is designed, so as to induce gene deletion, insertion or substitution by homeologous recombination at a target locus. Strategies to design targeting sequence for modifying a locus by homologous recombination are well known in the art. Any of such strategy may be employed to design the extrachromosomal targeting sequence as defined in the method of the present invention.

Said extrachromosomal targeting sequence may further be physically linked to one or more selectable markers for the selection of transformants. Such markers include for example, genes which confer phenotypic traits such as herbicide resistance, antibiotic resistance or disease resistance, or which confer some other recognizable trait such as male sterility, male fertility, grain size, colour, growth's rate, flowering time or ripening time.

For example, the extrachromosomal targeting sequence may comprise a gene or a portion thereof comprising respectively, at least: (i) the 3'end and the flanking intron junction-sequences from a first exon and (ii) the 5'end and the flanking intron junction-sequences from a second exon (consecutive or nonconsecutive to the first exon). Preferably, said targeting sequence comprises also, a selectable marker gene, inserted in the intronic sequences situated between the two exons. More preferably, said expression cassette is flanked by site-specific recombination sites, for example Lox sites.

Said extrachromosomal targeting sequence is introduced in the plant or cell thereof by suitable methods of transforming plants or plant cells, which are well known in the art and any of such methods may be employed to transform the plant or cell thereof according to the method of the present invention. According to the present invention, the extrachromosomal targeting sequence which is introduced in a plant or cell thereof integrates into the genome of the plant by homeologous recombination and is capable of being inherited by progeny thereof (stable transformation).

Said extrachromosomal targeting sequence may be introduced in the plant or cell thereof prior, concomitantly or subsequently to the transitory inactivation of MSH2. Preferably, said extrachromosomal targeting sequence is introduced concomitantly to said inactivation of MSH2. The inactivation of MSH2 may be performed according to any of the first or the second advantageous embodiment of the method of the present invention, as described above.

Preferably, the selectable marker gene is further deleted from the genome of the transgenic plant or cell thereof by a site-specific recombinase, Cre for example. Therefore, a recombinant plasmid encoding said site-specific recombinase operably linked to a promoter which is functional in plants is introduced in said transgenic plant or cell thereof by suitable methods of transforming plants or plant cells. The recombinase produced in the cells induces site specific recombination, which results in the deletion of the selectable gene marker.

According to a fourth advantageous embodiment of the method of the invention, it is a method for producing a hybrid plant or cell thereof, wherein:
- step a) comprises providing a plant in which MSH2 is inactivated during meiosis, and
- step b) comprises: crossing the recipient plant in a) with a donor plant having a genome comprising a sequence which has from 0.1 % to 10 % differences with a target sequence in the genome of said recipient transgenic plant, and maintaining the hybrid plant, under conditions allowing self-fertilization and production of offspring plant (progeny).

Said plant in step a) may be:
- a *msh2* knock-out mutant,
- a *msh2* variant (natural or recombinant (transgenic)) carrying an *msh2* allele which is functional during mitosis but not during meiosis. Examples of variants which are functional during mitosis but not during meiosis include transgenic plants wherein the *msh2* gene is under the control of the CaMV 35S promoter,
- a transgenic plant comprising a polynucleotide encoding, either a dominant negative mutant of MSH2, or a sense, antisense, ribozyme, or RNAi molecule which is similar or complementary to an *msh2* transcript, said polynucleotide being operably linked to a meiocyte specific promoter.

Preferably, step a) comprises providing: a transgenic plant comprising a polynucleotide encoding, either a dominant negative mutant of MSH2, or a sense, antisense, ribozyme, or RNAi molecule which is similar or complementary to an *msh2* transcript, said polynucleotide being operably linked to a meiocyte specific promoter, or a *msh2* variant (natural or recombinant (transgenic)) carrying an *msh2* allele which is functional during mitosis but not during meiosis.

The meiocyte specific promoter is for example, DMC 1 or Switch 1.

The hybrid plant resulting from homeologous recombination between the transgenic plant and the donor plant may be further identified on the evidence of a new genetic linkage of a desired trait or of a gene which contributes to a desired characteristic or trait.

Furthermore, when *msh2* is inactivated in step a), said method comprises an additional step d) of recovering MSH2 activity in the offspring. Said recovery may be performed by stable transformation of the F2 plants which have integrated the gene of interest from the donor plant, with a polynucleotide encoding MSH2, operably linked to a promoter which is functional in plants, and selection of the transformants expressing MSH2 (functional integration of the polynucleotide). Alternatively, said recovery is performed by crossing said F2 plants with a wild-type plant from the same species as the *msh2* knock-out mutant in step a) (backcross with the recipient species, and isolating the recombinants carrying a (wild-type) *msh2* allele.

This method allows the production of new hybrid plants or cells thereof comprising alleles of genes encoding desirable production or quality traits present in other species but which where unusable to breeders due to genetic isolation, but could be inserted at a desired locus by homeologous recombination.

The donor plant (B) is preferably from a species related to the recipient species (A) and carries a gene encoding a trait of interest. The interspecies hybrids formed (F1, A/B) from this interspecies crossing are identified in the next generation (F2), for example, by the appropriate application of genetic markers closely linked to the gene of interest and markers to loci in the homeologous region within the genome of the plant related to the donor species. Successive backcross of the F2 plant (carrying the gene of interest from the donor B), with the recipient plant A (non transgenic), allows the isolation of plants with the genetic background of A.

According to a fifth advantageous embodiment of the method of the invention, said targeting sequence has from 0.2% to 3% differences with said target sequence, preferably from 0.2% to 5%, more preferably from 0.2% to 7%.

According to a sixth advantageous embodiment of the method of the invention, it comprises a further step of selecting plants or plant cells progeny in which both target alleles have been modified by homeologous recombination.

The invention provides also, a dominant negative mutant of MSH2 comprising at least one mutation selected in the group consisting of: S493P, K542E, G670D, K671R and S672P, wherein the positions are indicated by reference to *P. patens* amino acid sequence SEQ ID NO: 2, which is encoded by the MSH2 cDNA sequence SEQ ID NO: 1. Knowing the mutations in *P. patens* MSH2 sequence, one skilled in the art will find easily the corresponding mutations in the MSH2 sequence from other organisms.

According to an advantageous embodiment of said dominant negative mutant, it is a plant variant, preferably a moss variant, for example a variant of *P. patens* sequence SEQ ID NO: 2.

The invention provides also a polynucleotide encoding said dominant negative variant of MSH2.

The invention provides also a recombinant vector, preferably an expression vector, comprising said polynucleotide.

The invention provides also a eukaryotic or prokaryotic cell which has been stably transformed with said polynucleotide or said recombinant vector.

The invention provides also a transgenic plant, stably transformed with a polynucleotide encoding a dominant negative variant of MSH2 as defined above, said polynucleotide being operably linked to an inducible promoter which is functional in plants.

According to an advantageous embodiment of said transgenic plant, the inducible promoter is a meiocyte specific promoter, as defined above.

According to another advantageous embodiment of said transgenic plant, it is a transgenic moss which may be derived, for example from *Physcomitrella patens.*

The invention provides also a RNAi targeting a sequence of at least 20 consecutive nucleotides situated between positions 950 and 1430 of a plant MSH2 cDNA, by reference to *P. patens* cDNA sequence SEQ ID NO: 1.

According to an advantageous embodiment of said RNAi, it is targeting the sequence from positions 950 to 1430.

According to another advantageous embodiment of said RNAi, it is a hairpin RNAi.

According to another advantageous embodiment of said RNAi, it is targeting a moss cDNA, for example *Physcomitrella patens* cDNA sequence SEQ ID NO: 1.

The invention provides also a polynucleotide construct designed for the production of said RNai, in a plant.

The invention provides also a recombinant vector comprising said polynucleotide.

The invention provides also a eukaryotic or prokaryotic cell which has been stably transformed with said polynucleotide or said recombinant vector.

The invention provides also a transgenic plant, stably transformed with a polynucleotide construct designed for the production of said RNAi in plants, said polynucleotide being operably linked to an inducible promoter which is functional in plants.

According to an advantageous embodiment of said transgenic plant, the inducible promoter is a meiocyte specific promoter, as defined above.

According to another advantageous embodiment of said transgenic plant, it is a transgenic moss which may be derived, for example from *Physcomitrella patens.*

The invention provides also a moss mutant or cell thereof, wherein at least one allele of the *msh2* gene has been disrupted (Knock-out mutant).

The invention provides also a transgenic moss or cell thereof, wherein the *msh2* gene is under the control of a repressible promoter which is functional in plants.

According to another advantageous embodiment of said transgenic or mutant moss, they are obtained from *Physcomitrella patens.*

Said dominant negative mutant, RNAi, and the derived polynucleotides, vectors, cells and mutant or transgenic plants including *Physcomitrella patens* transgenics are useful for performing the MSH2 inactivation in step a) of the method of the invention. They are also useful as hosts for the production of recombinant proteins as well as model to study homeologous recombination in plants.

The polynucleotides according to the present invention are provided in expression cassettes, wherein the polynucleotide is operably linked to at least one regulation element which is functional in a plant cell, so as to permit production or synthesis of the desired product (sense or antisense RNA, RNAi, protein). Examples of such regulation elements include promoter, intron and terminator sequences. Preferred promoters include: constitutive [ubiquitin (maize Ubi-1); actin (rice act1); cauliflower mosaic virus 35S (CaMV 35S); NOS)], inducible [Hsp17.3B, tetracycline repressible, glucocorticoïd inducible, tissue specific (protoplast: rice catB (Iwamoto et al., Plant Physiol. Biochem., 2004, 42: 241-249); meiocyte: DMCI (Klimyuk and Jones, Plant. J., 1997, 11: 1-14), Switch 1 (Mercier et al., Genes Dev., 2001, 15: 1859-1871)].

Generally, the expression cassette comprises a selectable marker for the selection of transformed cells, for example a gene which confers phenotypic traits such as herbicide resistance, antibiotic resistance (neomycin phosphotransferase II (NptII or NPTII); hygromycin phosphotransferase (HPT)) or disease resistance, or which confers some other recognizable trait such as male sterility, male fertility, grain size, colour, growth's rate, flowering time or ripening time.

Preferably, the expression cassette is further inserted in a vector.

The term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors.

A vector according to the present invention comprises, but is not limited to, a YAC (yeast artificial chromosome), a BAC (bacterial artificial), a phage, a phagemid, a cosmid, a bacterial vector *(Agrobacterium tumefaciens, Agrobacterium rhizogenes),* a viral vector, a plasmid, a RNA vector or a linear or circular DNA or RNA molecule which may consist of chromosomal, non chromosomal, semi-synthetic or synthetic DNA. In general, expression vectors of utility in recombinant DNA techniques are often in the form of "plasmids" which refer generally to circular double stranded DNA loops which, in their vector form are not bound to the chromosome. Large numbers of suitable vectors are known to those of skill in the art. Vectors for *Agrobacterium-*mediated gene transfer are well-known in the art and include binary vectors (plasmid or a Bac-vector) comprising a marker gene cloned between the border repeats of an artificial T region, by which transformed plant cells can be selected (For a review, see for example Hooykaas, P.J., "Plant Transformation", Encyclopedia of Life Sciences, 2001, pp. 1-6, Nature Publishing Group/www.els.net). Vectors for stable expression of RNAi are described in Wesley et al., The Plant Journal, 2001, 27: 581-590 and http:// www.pi.csiro.au/rnai/vectors.htm. For example, pHANNIBAL is designed for the production of hairpin RNA under the control of the CaMV 35S promoter and the OCS terminator; a fragment of at least 300 nucleotides from the target gene (target sequence) is cloned in the sense and antisense orientation on either side of the intron (PDK intron) to become the two arms of the hairpin.

The polynucleotides as defined in the present invention may be prepared by any method known by the man skilled in the art. For example, they are amplified by polymerase chain reaction with specific primers. The recombinant vectors comprising said polynucleotide may be obtained and introduced in a host cell by the well-known recombinant DNA and genetic engineering techniques. The transgenic plants are constructed, using methods well-known in the art, as described above, for example, by *Agrobacterium-*mediated gene transfer.

DNA manipulations are performed using classical methods, according to standard procedures as those described in: Current Protocols in Molecular Biology (Frederick M. AUSUBEL, 2000, Wiley and son Inc, Library of Congress, USA*)* and Molecular Cloning: A Laboratory Manual, Second Edition, (Sambrook et al, 1989, Cold Spring Harbor, New York: Cold Spring Harbor Laboratory Press*).*

Plant cell culture is performed using classical methods, according to standard procedures as those described in: Plant cell culture technology (Yeoman, M.M., 1986, Blackwell; Oxford (GBR*)).*

Plant cell transformation and transgenic plant production are performed using classical methods, according to standard procedures as those described in: Transgenic plant research (Lindsey, K., 1998, Harwood, Amsterdam (NDLR*).* Although transformation protocols may vary depending on the type of plant or plant cell (monocot or dicot) targeted, suitable methods of transforming plants or plant cells and producing transgenic plants are well known in the art and any of such methods as those described in Transgenic plant research (Lindsey, K., 1998, Harwood, Amsterdam (NDLR*),* may be employed to produce the modified cells and transgenic plants according to the invention. Transformation may be performed for example but not limited to using: cell fusion; chemically aided transfection, microprojectile bombardment, microinjection and electroporation of DNA; *Agrobacterium tumefaciens, Agrobacterium rhizogenes* and plant virus vectors mediated gene transfer. For reviews in such techniques, see for example: Hooykaas, P.J., "Plant Transformation", Encyclopedia of Life Sciences, 2001, pp. 1-6, Nature Publishing Group/www.els.net.

The crossing of plants and the selection of the hybrids are well known in the art and any of such methods as those described in: Genetic mechanisms for hybrid breeding (Kuck, U. W. G., 1995, Blackxell Wissenschafts-Verlag, Oxford (GBR*)),* may be employed to produce a hybrid plant according to the method of the present invention.

The present invention will be further illustrated by the additional description and drawings which follows, which refers to examples demonstrating the the role of MSH2 in plant DNA metabolism, as illustrated with *Ppmsh2* disruptants. It should be understood however that these examples are given only by way of illustration of the invention and do not constitute in anyway a limitation thereof.

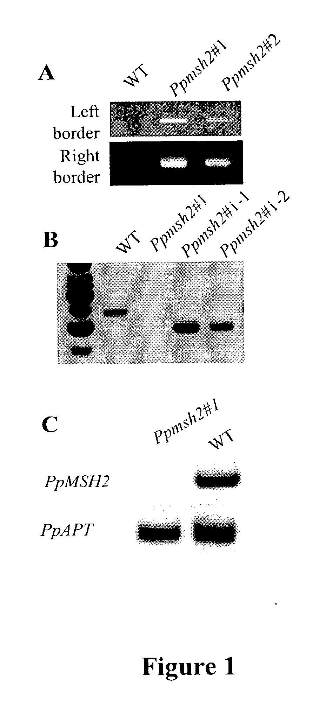

- Figure 1 illustrates the molecular analysis of *Ppmsh2* mutants, by comparison with wild type (WT). (A) PCR with a pair of primers, one primer specific for PpMSH2 sequence outside of the transformation construct (Left border or Right border) and the other primer specific for the resistance gene. (B) PCR with primers flanking recombination sites; *Ppmsh2#1-1* and *Ppmsh2#1-2* are mutants without the resistance gene resulting from *Ppmsh2#1* after Cre recombinase expression. (C) PpMSH2 transcripts.

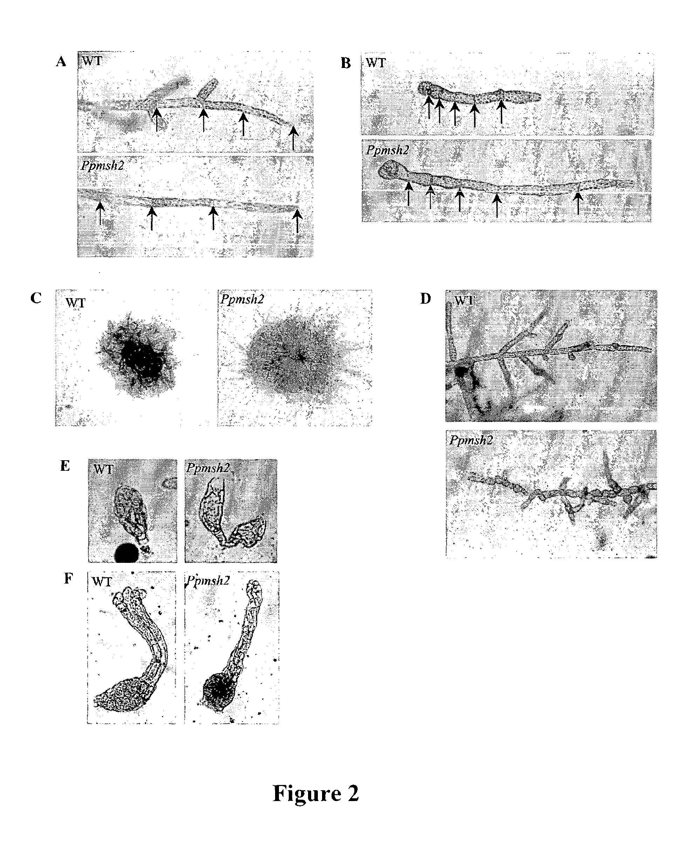

- Figure 2 represents the comparison of wild type and *Ppmsh2* mutant phenotypes: (A) Comparison of length of the three last cells of protonema filaments, arrows show the cell walls. (B) Number of cell division after eight days of protoplasts regeneration , arrows show the cell walls. (C) Wild type and *Ppmsh2* mutant colonies after nineteen days of development. (D) Protonema filaments of wild type and *Ppmsh2* mutant after one month of development. (E-F) sexual organs: (E) the male antheridia of wild type and *Ppmsh2* mutant, (F) the female archegonia of wild type and *Ppmsh2* mutant.

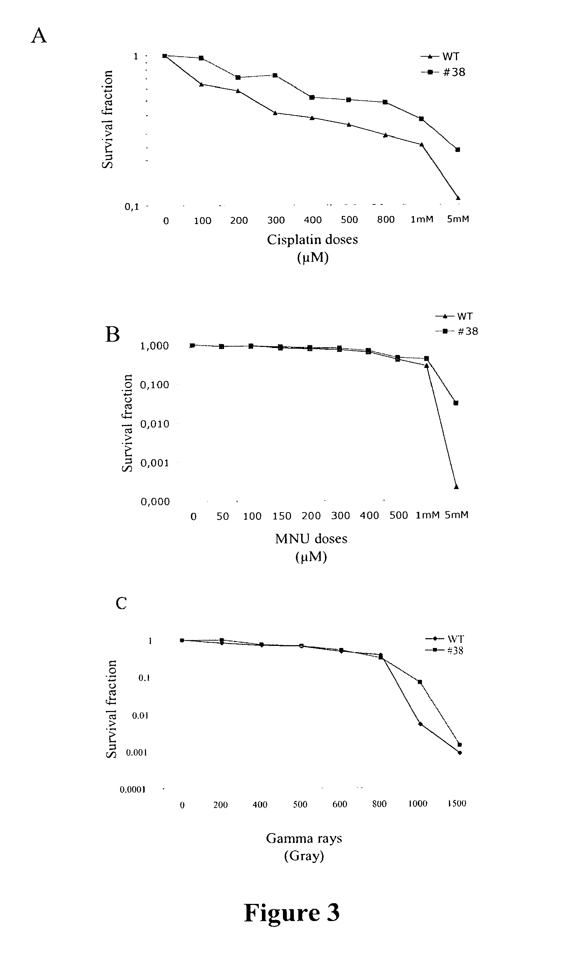

- Figure 3 illustrates the survival of wild type (WT) and *Ppmsh2* mutant, after genotoxic stresses. (A) Cisplatin. (B) N-methyl-N-nitrosourea (MNU). (C) Gamma radiation.

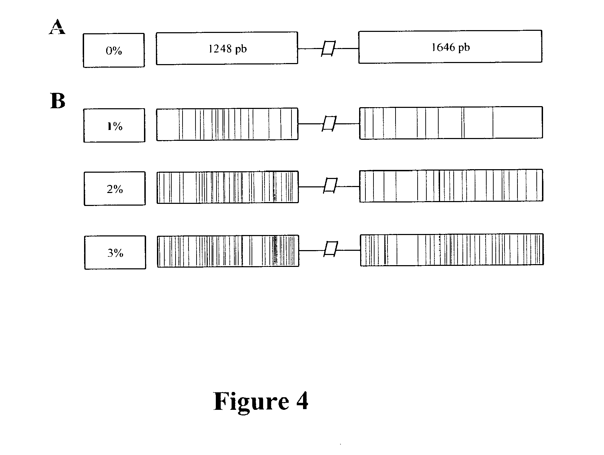

- Figure 4 represents gene targeting cassettes for homologous and homeologous recombination studies. (A) *PpAPT*-KO cassette in which *PpAPT* fragments are exactly identical to the locus *PpAPT* used as reporter gene. (B) Alignments of homeologous recombination cassettes, each mismatch between mutated *PpAPT* fragments and the original locus is indicated by a vertical line. The kanamycin gene resistance cassette is represented between both *PpAPT* fragments by an horizontal interrupted line

### Example 1: Targeted disruption of the msh2 gene in Physcomitrella patens

### 1) Material and Methods

### a) Plant material and culture conditions

*P. patens* tissues of the Gransden wild-type strain (Ashton and Cove, Mol. Gen. Genet., 1977, 154:87-95) were propagated on PpNH₄ medium, which corresponds to the minimal medium described by Ashton et al. (Planta, 1979, 144:427-435) supplemented with 2.7 mM NH₄ tartrate. Cultures were grown in 9 cm Petri dishes solidified with 0.7% Agar (BIOMAR) with a light regime of 16 h light /8 h darkness and a quantum irradiance of 80 µE.m⁻².sec⁻¹.

### b) Molecular cloning

Standard methods were used for all molecular cloning *(*Sambrook et al, 1989, Molecular Cloning: A Laboratory Manual, Second Edition, Cold Spring Harbor, New York: Cold Spring Harbor Laboratory Press*).*

### b₁) Cloning of Ppmsh2 gene

Using genomic *P. patens* DNA as starting template, a 6681 bp PCR fragment was amplified by performing a long-range PCR reaction with the pair of primers :
- PpMSH2#14: 5'-CGACTGGAGCGAGATTGTG-3' (SEQ ID NO: 8), and
- PpMSH2#15: 5'-CAACAGCTTGAAGACTGCG-3' (SEQ ID NO: 9).

The 6681 bp PCR fragment was subsequently cloned into pTopoBlunt (INVITROGEN).

### b₂) Isolation of Ppmsh2 mutants

Using genomic *P. patens* DNA as starting template, a 2368 bp PCR fragment was amplified by using the pair of primers:
- PpMSH2#4: 5'-CAATGTCACTGAGAGCAAAACG-3' (SEQ ID NO: 4; positions 2461 to 2482 on *Ppmsh2* gene), and
- PpMSH2#5: 5'-CGACCGAGCAGAGATCAGTGTG-3' (SEQ ID NO: 5; positions 4829 to 4808 on *Ppmsh2* gene).

The 2368 bp PCR fragment was subsequently cloned into the TA-cloning vector pCR^{®}II (INVITROGEN) to produce PpMSH2 plasmid. For the construction of PpMSH2-KO plasmid, the 5' fragment from *Not*I-DraI digestion and 3' fragment from *Sca*I*-Xho*I digestion of the PpMSH2 plasmid has been cloned in the pBSK35S-NPTII-lox vector, on both sides of the resistance cassette. The pBSK35S-NPTII-lox, which derives from the pBSK (STRATAGENE) contains a resistance cassette consisting of neomycin phosphotransferase gene *(NptII),* the expression of which is driven by the cauliflower mosaic virus 35S promoter (CaMV 35S). The antibiotic resistance cassette is in the same orientation in regard to the transcription unit of the *msh2* gene. In addition, the resistance cassette is flanked by lox sites (34 bp, Sauer B, Methods Enzymol., 1993, 225:890-900), to allow its elimination from the *Ppmsh2* mutants, by site-specific recombination between the two lox sites, in the presence of the Cre recombinase.

### c) Protoplasts isolation

Protoplasts were isolated from 6 days old protonema cultures by incubation for 40 min in 1% Driselase (FLUKA; # 44585) dissolved in 0,36 M mannitol. The suspension was filtered successively through 80 µm and 40 µm stainless steel sieves. Protoplasts were sedimented by low-speed centrifugation (600 g for 5 min at 20°C) and washed twice in 0.47 M mannitol. Protoplasts were then resuspended at 1.2×10⁻⁶ protoplasts/ml in MMM solution (0.47 M mannitol, 15 mM MgCl₂, and 0.1% MES pH 5.6).

### d) Transformation of protoplasts

Moss protoplasts (1.2×10⁶) were transformed as described previously (Schaefer et al, Mol. Gen. Genet., 1991, 226:418-424), by PpMSH2-KO plasmid digested with *Age*I and *Aat*II*.* Primary transformants were selected in the presence of 50 mg/L paramomycin (DUCHEFA, # P0141), which belongs to the kanamycin family of antibiotics. Small pieces of the protonema tissue (approximately 2 mm in diameter) of primary *P. patens* transformants were then transferred onto PP-NH₄ medium without selection for 15 days. The second round of selection for the isolation of stable transformants consisted in the transfer of small pieces of protonema tissue from the colonies of the previous transfer onto PpNH₄ medium containing 50 mg/L G418 (DUCHEFA, #G0175), which also belongs to the kanamycin family of antibiotics.

### e) Mutants screening

Moss DNA was extracted according to Edwards et al., NAR, 1991, 19: 1349. To screen stable disruptants of the *Ppmsh2* gene, the transformants were assayed for the incorporation of the resistance cassette into the *msh2* locus, by PCR with primers specific for the resistance cassette and primers specific for PpMSH2 sequence outside of the transformation construct.

The left border was analysed with the primers: CaMV 35S-Pro: 5'-TATTTTTGGAGTAGACAAGCGTGTCGT-3' (SEQ ID NO: 6; positions 229 to 203 on 35S-Npt II gene) and PpMSH2#4 (SEQ IDNO: 4), which amplify a 800 pb fragment.

The right border was analysed with the primers: PpMSH2≠5 (SEQ ID NO: 5) and CaMV-Ter: 5'-CCGCTTCCTCGTGCTTTACGGTAT-3' (SEQ ID NO: 7; positions 1462 to 1485 on 35S-Npt II gene), which amplify a 1800 bp fragment.

Doubled-stranded cDNAs corresponding to mRNAs expressed in six-day-old protonema cultures were obtained by using the SMART-PCR cDNA Synthesis Kit (CLONTECH).

### f) Cre recombinase expression in mutants

Transient expression of a constitutive Cre expression cassette (Albert et al., Plant J., 1995, 7:649-659) in protoplasts of a selected mutant was performed. Protoplasts were regenerated at low density on non-selective medium for two weeks, then fragments of single protoplast derived colonies were replicated on selective and non-selective medium. Loss of antibiotic resistance was scored after an additional week of growth, and replicates growing on non-selective medium were used for subsequent amplification and molecular analyses; 2 mutants were selected for further analysis.

### 2) Results

To examine the role of the Mismatch repair (MMR) system in *Physcomitrella patens,* the *Ppmsh2* gene was cloned and *Ppmsh2* disruptants were generated by homologous recombination using linear targeting construct (*Knock-out* ou KO cassette).

The *Ppmsh2* gene corresponds to the sequence SEQ ID NO: 3, wherein the positions of the exons are indicated in Table 1; the ATG and the stop codon correspond respectively to positions 351 and 6458.

**Table 1: msh2 gene exons positions**

| Number | Positions on *msh2* gene |
|---|---|
| I | 1 to 393 |
| II | 679 to 729 |
| III | 1064 to 1096 |
| IV | 1333 to 1619 |
| V | 1817 to 2095 |
| VI | 2241 to 2755 |
| VII | 3048 to 3603 |
| VIII | 3778 to 3889 |
| IX | 4081 to 4171 |
| X | 4352 to 4424 |
| XI | 4760 to 4871 |
| XII | 5023 to 5150 |
| XIII | 5438 to 5647 |
| XIV | 5870 to 6019 |
| XV | 6256 to 6681 |

Within 52 stable transformants, 11 strains *Ppmsh2* disruptants were identified by PCR, which correspond to a targeting efficiency of 21 %. By PCR, four disruptants showed a targeted replacement mediated by a double recombination event (Figure 1A). To demonstrate insertion of the targeted construct by homologous recombination at the *Ppmsh2* locus, disruption of the wild type targeted sequence was assessed by PCR with primers that flank the recombination sites. These results demonstrate integration of the KO cassette into the *Ppmsh2* locus for moss mutant analyzed (Figure 1B). In order to verify that the integration of the KO cassette into *msh2* gene indeed abrogated expression of the cognate mRNA, the steady-state amounts of mRNA was compared in the wild type and the KO mutant. Clearly, the disruption of the *msh2* gene by integration of the KO cassette resulted in the disappearance of the corresponding transcript in the KO mutant (Figure 1C). In order to obtain a simple deletion mutant for further analyses, one mutant was chosen to express the Cre recombinase in transient expression. A disrupted *Ppmsh2* locus with deletion of 518 bp (exon VII) was obtained, as confirmed this by PCR (Figure 1B).

### Example 2: Ppmsh2 mutants show developmental defects

### a) Early developmental defects

The *Ppmsh2* mutant regenerated protoplasts were distinguishable from wild type, as demonstrated by a more rapid development of mutant protoplasts in confluent colonies. The filaments size and cell divisions number were compared. Therefore, the length of the three last cells of protonema filaments from fourteen day-oid regenerated protoplasts were measured with a graduated ocular. The mean value for wild type is 4.2 +/- 1 and *Ppmsh2* mutant was 7.2 +/- 2 (Figure 2A and 2B). This difference is highly significant at a P value < 0.0001 with the Student test. This result shows that the length of the three last cells is longer in the mutants than in the wild type. Thus *Ppmsh2* mutant cells elongate 1.5 times more than the wild type cells. In order to evaluate cell division in the two backgrounds, the number of cell divisions was counted after eight days from regenerated protoplasts. The wild type mean cell divisions was 5 +/- 0.5 while for *Ppmsh2* mutant it was 6 +/- 0.8 (significant P < 0.0001 with the Student test) (Figure 2C and 2D). These results indicate that the mutant has a slight increase in cell division compared to the wild type. This observation evokes that *Ppmsh2* mutants could have an accelerated cell cycle. One hypothesis to explain this result would be a shorter arrest in cell cycle checkpoints. Indeed in mouse it has been shown that after DNA damage, the absence of MSH2 results in premature release from the early G2/M arrest following damage, with inability to activate properly the checkpoint kinase proteins, indicating that an active MSH2 is required for correct response to DNA damage in the G2 phase of the cell cycle, possibly connecting DSB repair to checkpoint signaling (Franchitto et al., Oncogene, 2003, 22: 2110-2120). Moreover in human, MMR is required for S-phase checkpoint activation (Brown et al., Nat. Genet., 2003, 33: 80-84). Consistent with these findings, it is supposed that PpMSH2 is required for efficient cell cycle arrest induced by DNA damage and could be a key component of acting DNA damage-activated checkpoints.

### b) Late developmental defects

*Ppmsh2* mutant colonies after few weeks have strong developmental problems. To highlight these phenotypes, similar size filaments were replicated from regenerated protoplasts on medium. After some days, WT filaments are entered in the transition from the filamentous juvenile to the leafy adult gametophyte. At the same time, *Ppmsh2* mutant colonies do not develop gametophores on filaments and protonema are more widespread. After three weeks, WT colonies had numerous gametophores well developed and *Ppmsh2* filaments just develop gametophores but only few compared to the wild type. Those gametophores are thinner and shorter. Wild type colony is more developed in three dimensions while *Ppmsh2* is more developed in two dimensions. Interestingly *Ppmsh2* filaments show round shaped cells probably due to aberrant cell divisions. After six weeks, mutant filaments start to become chlorotic. These observations indicate that *Ppmsh2* mutant seems to accumulate mutations during time.

### c) Sexual organs are not fertile

The later stage developmental of *Physcomitrella patens* is fertile sexual organs. In order to test whether developmental abnormalities seen at the protonema and gametophore stages were also present during sexual organs formation, antheridia and archegonia were observed in the two backgrounds. After ten weeks, wild type sexual organs are fully developed (Figure 2E, 2F) and this leads to the formation few weeks later to sporophyte. In all the *Ppmsh2* mutants tested sexual organs are abnormal (Figure 2E, 2F). In consequence the mutants show complete sterility. In order to test the possibility that sterility could be due to only once of the gamete, the mutant has been crossed by a male sterile line and by wild type. No spores have been produced on the male sterile line crossed by *Ppmsh2* mutant, and neither on the crossing with the wild type. Thus it can be concluded from those results that the *Ppmsh2* mutants are male and female sterile

One interpretation of this sterility is that *msh2* associated mutator phenotype might elevate the rate of mutations that causes problems to develop organs and specially complex organs like antheridia and archegonia. Similar phenotypes have been observed in *Arabidopsis thaliana msh2* mutant lines (Hoffman et al., 2004). After five generations plants showed abnormalities in morphology and development, fertility, germination efficiency, seed/silique development and seed set. In human and mice, mutations in *msh2* result in a greatly increased likelihood of developing certain types of tumors (Buermeyer et al., Annu. Rev. Genet., 1999, 33: 533-564). Like in other eukaryotes, *msh2* in moss during protoplast regeneration, growth, development and sexual organs formation, seems to be essential to maintain genetic stability and thus for species integrity. To investigate this hypothesis, spontaneous mutation rates in *Ppmsh2* mutants was evaluated, in comparison to the wild type.

### Example 3: Ppmsh2 mutants have a mutator phenotype

### 1) Material and methods

To assess mutator phenotype of the *Ppmsh2* mutants, the *PpAPT* gene was used as a reporter gene. Mutation in the *APT* gene confers resistance to 2FA (2FluoroAdenine) a toxic compound for cells. The number of mutations in the *PpAPT* gene reflects spontaneous mutations frequencies. Protoplasts of WT and *Ppmsh2* mutants were produced and spread after six days regeneration on PpNH₄ solid medium supplemented with 10 µM 2FA. After two weeks, the number of resistant colonies was counted.

### 2) Results

The strong developmental defects observed in *Ppmsh2* mutants could be related to an accumulation of spontaneous mutations, a phenotype already observed in *msh2* mutants of other organisms. To test this hypothesis, the effect of loss of the *PpMSH2* gene on mutation frequency of a reporter gene, the *PpAPT* gene, was investigated. Spontaneous mutations leading to loss of APT activity were selected on 2FA. For this purpose, a total of 10.10⁶ *Ppmsh2* mutant regenerated protoplasts aliquoted in 15 different inoculums and 3.10⁶ wild type regenerated protoplasts were plated in presence of 2-Fluoroadenine (2FA). The experiment was reproduced in 15 different inoculums in order to have a statistically relevant result. All the results given here are close to the median value. Whereas no 2FA-resistant colonies were seen in the WT culture, a mean of 27 resistant colonies per inoculum were present in the *Ppmsh2* culture. It can be concluded from these results, that spontaneous mutations rate is increased at least 130-fold in the *Ppmsh2* mutants compared to the wild type and thus that the *Ppmsh2* mutants show a strong mutator phenotype. Similar results were reported for MSH2 *of E. coli* (20 fold-increase; Schaaper et al., Proc. Natl. Acad. Sci., USA, 1987, 84: 6220-6224), *S pombe* (15 fold-increase; Rudolph et al., Mol. Cell. Biol., 1999, 19: 241-250), *S. cerevisiae* (200 fold-increase; Greene,et al., Genetics, 2001, 159: 65-75), *Caenorhabditis elegans* (30 to 70 fold-increase; Denver et al, Genetics, 2005, 170: 107-113), *Arabidopsis thaliana* (Hoffman et al., 2004), mouse (200 fold increase; de Wind et al., 1995) and human (several hundred fold increase; Malkhosyan, Mutat. Res., 1996, 316: 249-259). Moss mutation rate is in the same order of magnitude as other higher eukaryotes. On the other hand, in contrast to Msh2-deficient mouse cells, growth rate and development of moss *Ppmsh2* cells are affected, suggesting that the mutator phenotype interferes with different development pathways and later with cell viability. Based on these interpretations, it was concluded that PpMSH2 is essential for post-replicational MMR in moss, which is a role strongly preserved during evolution.

### Example 4: Different responses to genotoxic stresses in Ppmsh2 mutants

### 1) Material and methods

### a) Sensitivity to MNU / cisplatin

Cisplatin (cis-diamminedichloroplatinum(II); CALBIOCHEM), MNU (N-methyl-N-nitrosourea) and O⁶-bzGua (O⁶-benzylguanine) (SIGMA-ALDRICH) were dissolved in Dimethylsulfoxyde (DMSO) at concentration of 10 mM each for storage at -20°C and 25 mM for O⁶-bzGua immediately before use.

Wild type and *Ppmsh2* mutant protoplasts in liquid PpNH₄-mannitol medium were treated for 20 h with 0 - 5 mM MNU or cisplatin. During the whole procedure from 1 h prior to exposure to MNU, 25 µM O⁶-bzGua was present in the medium to inhibit removal of methyl groups from the O⁶ position of guanine by endogenous methyl-transferase activity. After 20 h in darkness, protoplasts were washed and spread on solid medium at high concentration for mutations frequency and low concentration for survival. After six days regeneration, protoplasts at low concentration were transferred on PpNH4 solid medium. After one week, survivals were counted. To test mutations frequency, plates with high concentration of protoplasts were transferred on PpNH4 solid medium supplemented with 10 µM 2FA. After two weeks, the number of resistant colonies was counted.

### b) Sensitivity to γ radiations / UV radiations

Protoplasts in liquid mannitol medium, were exposed to ionising radiation (1 Gy/s) by using a ¹³⁷Cs irradiator (IBL-637 CIS-Bio-International). After 20 h in darkness, protoplasts were spread on solid medium at high concentration for mutations frequency and low concentration for survival. Immediately spread on solid medium at high concentration for mutations and low concentration for survival, protoplasts were treated to UVB light (60 J/m²/S) from a 312 nm TFX lamp. The fluence was calculated with a UV-Elektronik dosimeter (GmbH). They were left in darkness for 20 h.

After 6 days regeneration, protoplasts at low concentration were transferred on PpNH₄ solid medium. After one week, survivals are counted. For mutations frequency, plates with high concentration of protoplasts were transferred on PpNH₄ solid medium supplemented with 10 µM 2FA. After two weeks, the number of resistant colonies was counted.

### 2) Results

MMR was suggested to recognize different DNA damages. It is believed that MMR mediates hypersensitivity to agents like radiations or chemicals that create DNA lesions (Fedier and Fink, Int. J. Oncol., 2004, 24: 1039-1047). It is predicted that loss of MMR will prevent cell death normally induced by genotoxic agents, thus conferring tolerance to these stresses. To determine whether *P. patens* cell containing mismatch repair enzyme gene defects were affected or not to different genotoxic stresses, *Ppmsh2* mutant protoplasts were treated with γ radiation and chemicals like cisplatin and MNU, and the survival and mutation rates were determined. Survival was calculated by the ratio of survival protoplasts after fifteen days regeneration after treatment to normal regeneration without treatment. Mutation rate was estimated in the same way as the estimate of spontaneous mutations frequency (example 3).

### a) γ-Radiation affect survival of Ppmsh2 mutants like the wild type, and mutation rate is not affected

Ionizing radiation induces a large variety of lesions in DNA, including not only strand breaks but also base damage and sugar damage (Hutchinson, Nucleic Acid. Res. Mol. Biol., 1985, 32: 115-154; Ward JF, Prog. Nucleic Acid Res. Mol. Biol., 1988, 35: 95-125). DNA damage such as oxidized bases (8-oxoguanine) induced by this radiation may be substrate for MMR (Fedier and Fink, 2004). In *Caenorhabditis elegans* X-rays DNA damage induced apoptosis is reduced and/or delayed in the absence of *msh-2,* but not eliminated (Degtyareva et al., 2002). Human hMutSα (mutS homolog) and *Drosophila spell* deficiency do not affect tolerance to γ-irradiation (Aquilina et al., Carcinogenesis., 1999, 20: 2317-2326; Flores and Engels, Proc. Natl. Acad. Sci. USA, 1999, 96: 2964-2969), while conflicting results were observed in mouse models (Fritzell, J.A. et al., Cancer Res., 1997, 57: 5143-5147; De Weese et al., Proc. Natl. Acad. Sci. USA, 1998, 95: 11915-11920 ; Franchitto et al., Oncongene, 2003, 22: 2110-2120). The effect of γ radiation on the survival of *Ppmsh2* mutant was compared with that of wild type (WT) protoplasts. The relative survival of these two genotypes was scored 15 days after exposure. Survival of wild type cells is affected by γ-radiation, proportionally with radiation dose. Survival of cells defective in MSH2 and their MSH2-proficient counterpart were affected in the same manner by the γ ray doses (Figure 3A). For both genotypes the LD₅₀ (Lethal Dose 50%) is reached at 600 Gray. These data indicate that *Ppmsh2* mutant cells have the same behavior as the WT under γ irradiation. Thus, in moss cells, loss of MSH2 does not affect the sensitivity to γ radiation, like in *Drosophila* and human cells.

### b) Ppmsh2 mutants are tolerant to cisplatin

Cisplatin is a DNA-damaging drug that forms bifunctional covalent adducts with DNA and is well-recognized mutagen in mammalian cells and affect their survival (Aebi, S. et al., Cancer Res., 1996, 56: 3087-3090). Experiments in *Escherichia coli* have shown that loss of MMR activity, because of mutation in *mutS,* was correlated with cisplatin resistance (Fram, R.J. et al., Mol. Pharmacol, 1985, 28: 51-55; Calmann, M.A. et al., Nucleic Acids Res., 2005, 33: 1193-1200). In contrast, Claij and te Riele have shown recently that, mouse embryogenic stem cells deficient for the MMR protein MSH2 respond in the same as wild-type cells following exposure to cisplatin (Claij and te Riele, Oncogene, 2004, 23: 260-266). In order to test cisplatin effect on mosses and the potential involvement of MMR system, the survival and mutation rate of wild type and *Ppmsh2* mutant regenerated protoplasts was compared after exposure to increasing concentration of cisplatin. Figure 3B shows that wild type survival is affected by cisplatin with a LD₅₀ at 250 µM. *Ppmsh2* cells were affected by cisplatin but they are resistant by two-fold in comparison to the wild type. *Ppmsh2* LD₅₀ is about 500 µM. All points of the survival curve are significantly different with the χ² test. These experiments show that the *Ppmsh2* deficient moss cells have a cisplatin tolerant phenotype, which could be a result from failure to efficiently execute cell death in response to cisplatin.

### c) Ppmsh2 mutants show a weak resistance to MNU

The cytotoxicity of methylating agents, such as MNU (N-methyl-N-nitrosourea) is largely caused by their ability to introduce O⁶-methylguanine (O₆₋meGua) into DNA (Humbert, et al., Carcinogenesis, -1999, 20: 205-214). Persistent DNA O⁶-meGua is normally lethal. Msh2-nullyzygous mouse cells showed a high level of MNU resistance (Fritzell et al., Cancer Res., 1997, 57: 5143-5147). Msh2-defective mouse cells were as resistant to MNU as repair-defective human cells (Humbert et al., 1999). In order to test moss sensitivity to methylation damage, the survival and mutation rates of wild type and *Ppmsh2* mutant cells were determined after exposure to increasing concentration of MNU. Wild type survival is affected by MNU treatment. The *Ppmsh2* mutant cells showed a slightly but significant better survival than the wild type (Figure 3D). The LD₅₀ is reached at 500 µM for both genotypes. These results show that *Ppmsh2* deficient moss cells have a weak resistance to MNU and indicate that a fraction of the MNU cytotoxicity in moss cells is dependent on MSH2 function.

### Example 5: Loss of suppression of recombination between diverged sequences in Ppmsh2 mutants

### 1) Material and methods

*Ppmsh2* gene involvement in homologous recombination has been studied by testing gene targeting efficiency at the locus of the *APT* reporter gene with homologous and homeologous sequences.

For the construction of PpAPT-KO, a 3995 bp fragment has been cloned in a pBlueScript KS vector (STRATAGENE) between *Xba*I and *EcoRI* restriction sites to give the plasmid PpAPT1 . In PpAPT1, the ATG codon is at 1217 bp from *Xba*I and the stop codon is at 881 bp from *EcoR*I. A 1102 bp *BamH*I fragment has been deleted by digestion of this plasmid and replaced by the hygromycin phosphotransferase gene *(HptII),* the expression of which was driven by the cauliflower mosaic virus 35S promoter (CaMV 35S) (Figure 4A).

To generate homeologous version of this disruption cassette, the APT fragment into the PpAPT1 vector was mutated by mutagenic PCR using the protocol described in Cadwell and Joyce, PCR Methods Appl., 1992, 2: 28-33. This method is based on the addition of MnCl₂ in the PCR mix to diminish the template specificity of the polymerase (Beckman et al., Biochemistry, 1985, 24: 5810-5817) and on an imbalance of dNTPs to promote misincorporation (Cadwell and Joyce, 1992). Different APT versions that contain until 3% of divergency with the original fragment, were generated. The *HptII* resistance gene was then introduced at the *BamH*I restriction site in every mutated vector, to select integration events (Figure 4B).

After transformation of WT and *Ppmsh2* mutants protoplasts with homologous and homeologous APT disruption vectors, stable transformants, were selected. Then, small pieces of protonema tissue (approximately 2 mm in diameter) from stable transformants were transferred onto PpNH₄ medium containing 10 µM of 2-Fluoroadenine, to detect APT gene targeting events. Gene targeting frequency is calculated by the ratio of APT mutants to the number of stable transformation events (i.e., stables hygromycin resistant colonies).

### 2) Results

*Ppmsh2* gene involvement in homologous recombination (HR) has been studied by testing gene targeting (GT) efficiency at the locus of the *APT* reporter gene with homologous and homeologous sequences. Wild type and *Ppmsh2* cells were transformed with APT vectors that contain 0%, 1%, 2% and 3% divergences respectively compared to the *PpAPT* locus. 2FA resistant clones were isolated and counted. To obtain the gene targeting frequency, the total number of stable transformants divided the number of targeting events. Stable transformants are the ones that have integrated the APT vector in their genome (targeted or not to the *PpAPT* locus). The transformation efficiency was estimated by calculating the ratio between stables and all resistant clones from the first selection round.

**Table 2: Transformation efficiency (TE) and gene targeting frequency (GT) in wild type and Ppmsh2 mutant (number of regenerants are comparable)**

| | **WT** | | ***Ppmsh2*** | |
|---|---|---|---|---|
| **Divergences** | **TE** | **GT** | **Stables** | **GT** |
| **0%** | 20% (203/998) | 44% (90/204) | 30% (281/924) | 24% (68/278) |
| **1%** | *12.5% (81*/*648)* | 14% (11/81) | *18% (106*/*600)* | *25% (27*/*106)* |
| **2%** | *7% (46*/*657)* | 9% (4/46) | *12%(8*/*17)* | *25% (1*/*4)* |
| **3%** | 4% (128/2902) | 2% (2/124) | 10% (98/943) | 30% (29/96) |

A strong decrease, five-fold, in transformation efficiency (number of stable transformants / number of resistants from the first selection), was observed, with homeologous fragment in the wild type background. In the mutant, the level of transformation efficiency with homologous cassette is slightly higher compared to the wild type (1.5-fold) and the decrease in transformation efficiency with homeologous cassette is weaker (3-fold).

Interestingly, a slight but statistically significant decrease (1.8-fold) in homologous recombination frequency was observed for the mutant compared to wild type (P value = 0.001).

Wild type recombination frequency decreased of twenty-two-fold with 3% mismatch (significant with χ² test, P value < 0.00001), whereas in *Ppmsh2* mutant, recombination with homeologous fragments was as efficient as with homologous fragment (non significantly different, P value = 0.40). This experiment demonstrates that lack of *Ppmsh2* allows the targeting at a locus with homeologous sequences up to 3%.

These results demonstrate that DNA sequence identity is an important determinant of the efficiency of gene replacement in moss. In addition, lack of *Ppmsh2* allows targeting to a locus with homeologous sequences up to 3% as efficiently as with perfectly identical sequences. This result demonstrates that *Ppmsh2* mutants have lost the barrier to recombination between homeologous sequences. It can be concluded that PpMSH2 is involved in preventing homologous recombination between diverged DNA. The extent to which *P. patens* homologous recombination, acting on both identical and non-identical substrate, is enhanced by mutation of *msh2* is comparable with that seen in E. *coli* (Rayssiguier et al., 1989), in Trypanosome (Bell, J.S., and McCulloch, R., J. Biol. Chem., 2003, 278: 45182-45188), in yeast (Negritto et al., 1997) and in mouse cells (de Wind et al., 1995; Abuin et al., 2000, Elliott and Jasin, 2001), suggesting that this role of MSH2 is strongly preserved during evolution.

While the frequency of recombination between mismatched DNA fragment and genomic target sequences was the same as the frequency of recombination between identical sequences in the *Ppmsh2* defective cells, it was surprinsing to find that the frequency of recombination between identical DNA was decreased 1,8-fold relative to the wild type homologous recombination frequency. In mouse, data on *msh2* mutants are consistent with the decrease of gene targeting efficiency with homologous fragment relative to wild type observed in *Physcomitrella patens,* and is in the same order of magnitude (1.5-fold) (de Wind et al., 1995). In this organism, to calculate gene targeting efficiency de Wind *et al.* divided homologous recombination events by total number of G418^{R} colonies. This calculation method is the same as above, in contrasts to yeast where recombination rate is calculated by dividing the number of targeted events by the number of viable cells. With this calculation method, in yeast the frequency of recombination with homologous fragment is higher in the *msh2* mutant than in the wild type (over 40-fold in Negritto, 1997; 2.7-fold in Datta, 1997). Conversely, one study reported the opposite result, they show that *msh2* mutation lowers the incidence of homologous recombination of a linear DNA fragment (Saparbaev et al., Genetics, 1996, 142: 727-736). If this method is applied to the results obtained in *Physcomitrella patens,* a slight (1.4-fold) but statistically significant (P = 0.007) increase in mutants recombination rate relative to wild type *(Ppmsh2:* 1.3x10⁻⁵ and WT: 9.10⁻⁴), is observed.

A closer examination of the hereabove homologous recombination results in *Physcomitrella patens,* allows noticing that the increase in gene targeting frequency of wild type compared to the mutant (1.7-fold) is compensated by the decrease observed in transformation frequency of wild type compared to the mutant (1.8-fold). Interestingly, this observation could demonstrate that gene targeting efficiency with homologous fragment is not effectively decreasing in *Ppmsh2* mutants, but the number of all integrations is increasing with lack of *Ppmsh2* function, therefore the number of illegitimate integrations. These results could indicate that MSH2 in *P. patens* is probably involved in mechanisms that regulate integrations in the genome but not only at the homologous recombination level but at the illegitimate recombination too.

## Claims

1. A method for producing a hybrid or transgenic plant, or cell thereof, comprising at least the steps of:
a) inactivating transitorily MSH2 in a plant or cell thereof,
b) introducing in said plant or cell thereof, a targeting sequence having from 0.1 % to 10 % differences with a target sequence in the genome of said plant or cell thereof, under conditions allowing homeologous recombination to occur between said target and targeting sequences, and
c) identifying the hybrid or transgenic plant or cell thereof, in which genomic integration of said targeting sequence as occurred through homeologous recombination with said target sequence.

2. The method of claim 1, wherein said transitory inactivation of MSH2 comprises the introduction of an MSH2 inhibitor in said plant or cell thereof.

3. The method of claim 2, wherein the MSH2 inhibitor is a drug selected in the group consisting of: cadmium and iododeoxyuridine.

4. The method of claim 2, wherein said inhibitor is a MSH2 dominant negative mutant comprising at least a mutation selected in the group consisting of: S493P, K542E, G670D, K671R and S672P, by reference to MSH2 sequence SEQ ID NO: 2.

5. The method of claim 1, wherein said transitory inactivation of MSH2 comprises the transitory inhibition of *msh2* gene expression in said plant or cell thereof.

6. The method of claim 5, wherein said transitory inhibition is mediated by a RNAi molecule targeting a sequence of at least 20 consecutive nucleotides situated between positions 950 and 1430 of MSH2 cDNA, by reference to SEQ ID NO: 1.

7. The method of claim 6, wherein said RNAi targets the sequence from positions 950 to 1430.

8. The method of claim 6 or claim 7, wherein said RNAi is a hairpin RNAi.

9. The method of claim 5, wherein said inhibition comprises providing a plant selected in the group consisting of: a *msh2* knock-out mutant and a *msh2* variant carrying an allele which is either functional during mitosis but not during meiosis, or under the control of an inducible promoter which can be switch off.

10. The method of claim 9, which comprises an additional step d) of recovering MSH2 activity in said hybrid or transgenic plants obtained in c).

11. The method of anyone of claims 1 to 10, wherein for producing a transgenic plant or cell thereof, said targeting sequence which is introduce in step b) is an extrachromosomal nucleic acid molecule.

12. The method of anyone of claims 1, 2 and 4 to 11, wherein for producing a hybrid plant or cell thereof:
- step a) comprises providing a plant in which MSH2 is inactivated during meiosis, and
- step b) comprises: crossing the recipient plant in a) with a donor plant having a genome comprising a sequence which has from 0.1. % to 10 % differences with a target sequence in the genome of said recipient transgenic plant, and maintaining the hybrid plant, under conditions allowing self-fertilization and production of offspring plant.

13. The method of claim 12, wherein the plant in step a) is a *msh2* knock-out mutant or a *msh2* variant carrying an *msh2* allele which is functional during mitosis but not during meiosis.

14. The method of claim 12, wherein the plant in step a) is a transgenic plant comprising a polynucleotide encoding, either a dominant negative mutant of MSH2, or a sense, antisense, ribozyme, or RNAi molecule which is similar or complementary to an *msh2* transcript, said polynucleotide being operably linked to a meiocyte specific promoter.

15. The method of claim 13, which comprises an additional step d) of recovering MSH2 activity in the offspring.

16. The method of claim 12, wherein the transgenic and donor plants are from different species.

17. The method of anyone of claims 1 to 16, wherein said targeting sequence has from 0.2 % to 3 % differences with said target sequence, preferably from 0.2 % to 5 %, more preferably from 0.2 % to 7 %.

18. The method of anyone of claims 1 to 17, comprising a further step of selecting plants or plant cells progeny, in which both target alleles have been modified by homeologous recombination.

19. A dominant negative mutant of MSH2 comprising at least one mutation selected in the group consisting of: S493P, K542E, G670D, K671R and S672P, wherein the positions are indicated by reference to SEQ ID NO: 2.

20. The mutant of claim 19, which is a mutant of *P. patens* SEQ ID NO: 2.

21. A polynucleotide encoding the mutant of claim 19 or claim 20.

22. A RNAi targeting a sequence of at least 20 consecutive nucleotides situated between positions 950 and 1430 of a plant MSH2 cDNA, by reference to SEQ ID NO: 1.

23. The RNAi of claim 22, which targets the sequence from positions 950 to 1430.

24. The RNAi of claim 22 or claim 23, which is a hairpin RNAi.

25. A polynucleotide designed for the production of the RNAi of anyone of claims 22 to 24, in a plant.

26. A recombinant vector comprising the polynucleotide of claim 21 or claim 25.

27. A cell stably transformed with the polynucleotide of claim 21 or claim 25, or the vector of claim 26.

28. A transgenic plant, stably transformed with the polynucleotide of claim 21 or claim 25, said polynucleotide being operably linked to an inducible promoter which is functional in plants.

29. The transgenic plant of claim 28 which is a transgenic moss.

30. The transgenic plant of claim 28 or claim 29, wherein said inducible promoter is a meiocyte specific promoter.

31. A transgenic moss or cell thereof, wherein the *msh2* gene is under the control of a repressible promoter which is functional in plants.

32. A moss mutant or cell thereof, wherein at least one allele of the *msh2* gene has been disrupted.

33. The transgenic or mutant moss of anyone of claims 29 to 32, which is obtained from *Physcomitrella patens.*
